# EUROPEAN PATENT APPLICATION

(11) **EP 4 039 687 A1**
(43) Date of publication of application: **10.08.2022**
(21) Application number: 20872471.6
(22) Date of filing: 01.09.2020
(51) Int. Cl.: C07D 487/04, C07D 487/22, C07D 487/14, A61K 31/519, A61P 25/28, A61P 9/10

(54) **TETRACYCLIC PYRIMIDINONE COMPOUND, PREPARATION METHOD THEREFOR, AND COMPOSITION AND USE THEREOF**

(30) Priority: 30.09.2019 CN 201910939482; 10.10.2019 CN 201910959237
(71) Applicant: Neusco Biotech Limited, Shanghai 201321 (CN)
(72) Inventor: XIAO, Qiong, Shanghai 201321 (CN); GU, Zhenghua, Shanghai 201321 (CN); HU, Youhong, Shanghai 201321 (CN)
(74) Representative: Bandpay & Greuter
(86) International application number: PCT/CN2020/112802
(87) International publication number: WO 2021/063145

(57) **Abstract**

Disclosed is a tetracyclic pyrimidinone compound represented by formula (I) or a pharmaceutically acceptable salt thereof. The compound having a structure represented by formula (I) is a new Lp-PLA2 inhibitor usable for treatment of neurodegenerative-related diseases, such as Alzheimer's disease (AD), glaucoma and age-related macular degeneration (AMD), or of cardiovascular diseases including atherosclerosis.

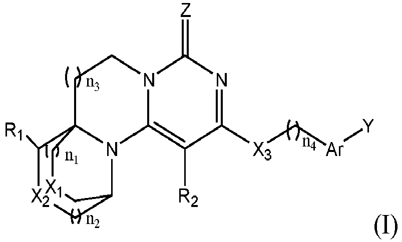

## Description

### TECHNICAL FIELD

The present disclosure belongs to the field of medicine, and specifically relates to a tetracyclic pyrimidinone compound, a preparation method therefor, and a composition thereof, and use thereof in medicine.

### BACKGROUND

Lipoprotein-associated phospholipase A2 (Lp-PLA2) is a member of the phospholipase A2 superfamily (Dennis EA, Cao J, Hsu YH, Magrioti V, Kokotos G. Chem Rev. 2011, 111, 6130-6185). It is mainly secreted by monocytes, macrophages, T lymphocytes, and chief cells (Stafforini DM, Elstad MR, McIntyre TM, Zimmerman GA, Prescott SM. J Biol Chem. 1990, 265: 9682-9687; Nakajima K, Murakami M, Yanoshita R, Samejima Y, Karasawa K, Setaka M, Nojima S Kudo I. J Biol Chem. 1997, 272, 19708-19713). Phosphatidylcholine sn-2 esters are produced during oxidation of low-density lipoprotein (LDL), and Lp-PLA2 is responsible for hydrolysis of oxidatively modified phosphatidylcholine sn-2 esters, and then producing oxidized fatty acids and lysophosphatidylcholine (LysoPC) (Caslake MJ, Packard CJ, Suckling KE, Holmes SD, Chamberlain P, Macphee CH. Atherosclerosis. 2000, 150, 413-419; MacPhee CH, Moores KE, Boyd HF, Dhanak D, Ife RJ, Leach CA, Leake DS, Milliner KJ, Patterson RA, Suckling KE, Tew DG, Hickey DM. Biochem J. 1999, 338, 479-487). Both oxidized fatty acids and LysoPC play roles in activating macrophages, increasing oxidative stress, affecting T lymphocyte function, and inducing inflammatory responses (Quinn MT, Parthasarathy S, Steinberg D. Proc Natl Acad Sci USA. 1988, 85, 2805-2809). LysoPC has been reported to induce the release of multiple cytotoxic inflammatory cytokines (Shi, et al, Atherosclerosis, 2007, 191, 54-62). In addition, LysoPC is also involved in leukocyte activation, induction of apoptosis, and mediation of endothelial dysfunction (Wilensky et al, Current Opinion in Lipidology, 2009, 20, 415-420).

It has been reported that the Lp-PLA2 level of plasma is associated with cardiovascular diseases (Fitzpatrick AL, Irizarry MC, Cushman M, Jenny NS, Chi GC, Koro C. Atherosclerosis. 2014, 235, 384-391), diabetic macular edema (DME) (Staurenghi G, Ye L, Magee MH, Danis RP, Wurzelmann J, Adamson P, McLaughlin MM, Darapladib DMESG. Ophthalmology. 2015, 122, 990-996), and prostate cancer (Bertilsson H, Tessem MB, Flatberg A, Viset T, Gribbestad I, Angelsen A, Halgunset J. Clin Cancer Res. 2012, 18, 3261-3269).

Alzheimer's disease (AD) is a chronic neurodegenerative disease that causes cognitive decline, mood swing, irreversible memory loss, disorientation, language impairment, and loss of self-protection (Hardy J, et al. Science 2002, 297, 353-356). Alzheimer's disease typically begins slowly and gets progressively worse over time, which is responsible for 60 to 70 percent of dementia cases and affects about 6 percent of the population over the age of 65. AD patients will gradually withdraw from family and society, become more and more dependent on help, and eventually progress to death. AD is one of the most costly diseases in developed countries, so as in other countries. Especially as aging becomes an important social issue, the costs will increase dramatically. There is no doubt that AD is a complex disease involving multiple factors. Although the pathogenesis of AD has not been fully elucidated, it is clear that several factors are involved in the development and progression of the disease, including aggregated tau proteins and Aβ peptides, oxidative stress and neuroinflammation (Echeverria V, Yarkov A, Aliev G. Prog Neurobiol. 2016, 144, 142-157). Current AD drug discovery focuses mainly on the targets of Aβ amyloidosis and tau (Chiang K, Koo EH. Annu Rev Pharmacol Toxicol. 2014, 54, 381-405; Awasthi M, Singh S, Pandey VP, Dwivedi UN. J Neurol Sci. 2016, 361, 256-271). However, despite reliable preclinical data, results from late-stage clinical trials have so far failed to demonstrate the clinical efficacy. These disappointing results suggest that other neuropathological mechanisms, such as oxidative stress and neuroinflammation, may have to be explored for AD treatment.

Elevated levels of Lp-PLA2 in plasma increase the risk of dementia, including AD (Van Oijen, et al. Annals of Neurology, 2006, 59,139). Vascular dementia and mixed dementia as well as high oxidative LDL levels have been found in AD patients (Maher-Edwards G, De' Ath J, Barnett C, Lavrov A, Lockhart A, Alzheimer's & Dementia: Translational Research & Clinical Interventions. 2015, 1, 131-140; Kassner et al. Current Alzheimer Research, 2008, 5, 358-366; Dildar, et al., Alzheimer Dis Assoc Disord, 24, April-June ( 2010); Sinem, et al. Current Alzheimer Research, 2010, 7, 463-469). Neuroinflammation and a plurality of up-regulated inflammatory cytokines have also been found in AD patients (Colangelo, et al., Journal of Neuroscience Research, 2002, 70, 462-473; Wyss-Coray, Nature Medicine, 2006, 12, Sept.).

In view of all of these findings, Lp-PLA2 is a potential target for the treatment of AD, which is further confirmed by the clinical results of Lp-PLA2 inhibitor Rilapladib in AD patients (Maher-Edwards G, De'Ath J, Barnett C, Lavrov A, Lockhart A, Alzheimer's & Dementia: Translational Research & Clinical Interventions. 2015, 1, 131-140).

Glaucoma and age-related macular degeneration (AMD) are retinal neurodegenerative diseases. Buschini, *et al.* have reported that inflammation, including TNF-α signaling, may play an important role in the pathogeneses of glaucoma and AMD (Buschini et al, Progress in Neurobiology, 2011, 95, 14-25; Tezel, Progress in Brain Research, vol. 173, ISSN0079-6123, Chapter 28). In addition, Shi, *et al.* have demonstrated that the Lp-PLA2 inhibitor can block the release of inflammatory cytokines (Shi, et al, Atherosclerosis, 2007, 191, 54-62). Inhibition of Lp-PLA2 is a potential therapy for glaucoma and AMD.

Many Lp-PLA2 inhibitors have been reported, including β-lactams (Tew DG, Boyd HF, Ashman S, Theobald C, Leach CA. Biochemistry. 1998, 37, 10087-10093), oximes (Jeong TS, Kim MJ, Yu H, Kim HS, Choi JK, Kim SS, Lee WS. Bioorg Med Chem Lett. 2005, 15, 1525-1527; Jeong HJ, Park YD, Park HY, Jeong IY, Jeong TS, Lee WS. Bioorg Med Chem Lett. 2006, 16, 5576-5579), amides of xanthuric acids (Lin EC, Hu Y, Amantea CM, Pham LM, Cajica J, Okerberg E, Brown HE, Fraser A, Du L, Kohno Y, Ishiyama J, Kozarich JW, Shreder KR. Bioorg Med Chem Lett. 2012, 22, 868-871; Hu Y, Lin EC, Pham LM, Cajica J, Amantea CM, Okerberg E, Brown HE, Fraser A, Du L, Kohno Y, Ishiyama J, Kozarich JW, Shreder KR. Bioorg Med Chem Lett. 2013, 23, 1553-1556), and urethane (Nagano JM, Hsu KL, Whitby LR, Niphakis MJ, Speers AE, Brown SJ, Spicer T, Fernandez-Vega V, Ferguson J, Hodder P, Srinivasan P, Gonzalez TD, Rosen H, Bahnson BJ, Cravatt BF. Bioorg Med Chem Lett. 2013, 23, 839-843).

The Lp-PLA2 inhibitor Darapladib has been reported as a potential therapy against atherosclerosis and DME (Magrioti V, Kokotos G. Expert Opin Ther Pat. 2013; 23: 333-344).

### SUMMARY

The present inventors have found that Lp-PLA2 inhibitors have a significant role in treatment of neurodegenerative-related diseases such as Alzheimer's disease (AD), glaucoma and age-related macular degeneration (AMD), or cardiovascular diseases including atherosclerosis. To this end, the present inventors are dedicated to developing a brand new Lp-PLA2 inhibitor, i.e., tetracyclic pyrimidinone compound.

The tetracyclic pyrimidinone compound is a compound having a structure represented by formula (I), 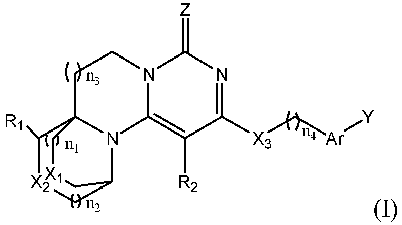 or a pharmaceutically acceptable salt thereof, wherein:
n₁, n₂, n₃, and n₄ are each independently 0, 1, or 2;
R¹ and R² are each independently selected from: -H, hydroxyl, cyano, halogen, alkyl, deuteroalkyl, hydroxyalkyl, haloalkyl, cycloalkyl, and alkoxyl;
X₁, X₂, and X₃ are each independently selected from: alkylene, -O-, -S-, or -NR'-;
R' is selected from: -H, alkyl, deuteroalkyl, or cycloalkyl;
Ar is arylene or heteroarylene; and hydrogen atoms in the arylene or heteroarylene are optionally substituted by one or more substituents that are each independently selected from: halogen, alkyl, haloalkyl, alkoxyl, haloalkoxyl, deuteroalkyl, deuteroalkoxyl, hydroxyl, hydroxyalkyl, cyano, amino, monoalkyl- or dialkyl-substituted amino, nitro, carboxyl, aldehyde, cycloalkyl, heterocyclyl, aryl, or heteroaryl;
Y is -H, halogen, alkyl, haloalkyl, haloalkoxyl, cycloalkyl, alkoxyl, deuteroalkyl, deuteroalkoxyl, -OAr', -SAr', -NH-Ar', -NMe-Ar', -NR", or -R‴-Ar';
Ar' is selected from aryl or heteroaryl; and hydrogen atoms in the aryl or heteroaryl are optionally substituted by one or more substituents that are each independently selected from: halogen, alkyl, haloalkyl, alkoxyl, hydroxyl, hydroxyalkyl, haloalkoxyl, deuteroalkyl, deuteroalkoxyl, cyano, amino, nitro, carboxyl, aldehyde, cycloalkyl, heterocyclyl, aryl, or heteroaryl;
R" is alkyl;
R‴ is alkylene; and
Z is O or S;
optionally, wherein:
   n₁, n₂, n₃, and n₄ are each independently 0, 1, or 2;
   R¹ and R² are each independently selected from: -H, hydroxyl, cyano, halogen, alkyl, deuteroalkyl, hydroxyalkyl, haloalkyl, cycloalkyl, and alkoxyl;
   X₁, X₂, and X₃ are each independently selected from: alkylene, -O-, -S-, or -NR'-,
   R' is selected from: -H, alkyl, deuteroalkyl, or cycloalkyl;
   Ar is arylene or heteroarylene; and hydrogen atoms in the arylene or heteroarylene are optionally substituted by one or more substituents that are each independently selected from: halogen, alkyl, haloalkyl, alkoxyl, haloalkoxyl, hydroxyl, hydroxyalkyl, cyano, amino, monoalkyl- or dialkyl-substituted amino, nitro, carboxyl, aldehyde, cycloalkyl, heterocyclyl, aryl, or heteroaryl;
   Y is -H, halogen, alkyl, haloalkyl, haloalkoxyl, cycloalkyl, alkoxyl, -OAr', -SAr', -NH-Ar', -NMe-Ar', -NR", or -R‴-Ar';
   Ar' is selected from aryl or heteroaryl; and hydrogen atoms in the aryl or heteroaryl are optionally substituted by one or more substituents that are each independently selected from: halogen, alkyl, haloalkyl, alkoxyl, hydroxyl, hydroxyalkyl, haloalkoxyl, cyano, amino, nitro, carboxyl, aldehyde, cycloalkyl, heterocyclyl, aryl, or heteroaryl;
   R" is alkyl;
   R‴ is alkylene; and
   Z is O or S.

Optionally, halogen atoms in the "halogen", "haloalkyl", and "haloalkoxyl" are each independently selected from F, Cl, Br, or I;
optionally, alkyls in the "alkyl", "deuteroalkyl", "deuteroalkoxyl", "hydroxyalkyl", "haloalkyl", "haloalkoxyl", "alkoxyl", "monoalkyl- or dialkyl-substituted amino" are each independently C₁-C₁₀ linear or branched alkyl; optionally, C₁-C₇ linear or branched alkyl; optionally, C₁-C₄ linear or branched alkyl; and optionally, selected from methyl, ethyl, n-propyl, isopropyl, n-butyl, isobutyl, tert-butyl, sec-butyl, n-pentyl, 1-methylbutyl, 2-methylbutyl, 3-methylbutyl, isopentyl, 1-ethylpropyl, neopentyl, n-hexyl, 1-methylpentyl, 2-methylpentyl, 3-methylpentyl, isohexyl, 1,1-dimethylbutyl, 2,2-dimethylbutyl, 3,3-dimethylbutyl, 1,2-dimethylbutyl, 1,3-dimethylbutyl, 2,3-dimethylbutyl, 2-ethylbutyl, n-heptyl, 2-methylhexyl, 3-methylhexyl, 2,2-dimethylpentyl, 3,3-dimethylpentyl, 2,3-dimethylpentyl, 2,4-dimethylpentyl, 3-ethylpentyl, or 2,2,3-trimethylbutyl;
optionally, the "alkylenes" are each independently C₁-C₁₀ linear or branched alkylene; optionally, C₁-C₇ linear or branched alkylene; optionally, C₁-C₅ linear or branched alkylene; optionally, selected from methylene, ethylene, n-propylidene, isopropylidene, n-butylidene, isobutylidene, tert-butylidene, sec-butylidene, n-pentylidene, 1-methylbutylidene, 2-methylbutylidene, 3-methylbutylidene, isopentylidene, 1-ethylpropylidene, neopentylidene, n-hexylidene, 1-methylpentylidene, 2-methylpentylidene, 3-methylpentylidene, isohexylidene, 1,1-dimethylbutylidene, 2,2-dimethylbutylidene, 3,3-dimethylbutylidene, 1,2-dimethylbutylidene, 1,3-dimethylbutylidene, 2,3-dimethylbutylidene, 2-ethylbutylidene, n-heptylidene, 2-methylhexylidene, 3-methylhexylidene, 2,2-dimethylpentylidene, 3,3-dimethylpentylidene, 2,3-dimethylpentylidene, 2,4-dimethylpentylidene, 3-ethylpentylidene, or 2,2,3-trimethylbutylidene;
optionally, the "cycloalkyl" is C₃-C₁₀ monocyclic or bicyclic cycloalkyl, optionally C₃-C₇ monocyclic cycloalkyl, and optionally cyclopropyl, cyclobutyl, cyclopentyl, cyclohexyl, or cycloheptyl;
optionally, the "heterocyclyl" is 3- to 10-membered non-aromatic heterocyclic ring containing 1, 2, or 3 heteroatoms selected from N, O, and S on the ring, optionally, the heterocyclic ring is a 3- to 10-membered non-aromatic ring containing 1 or 2 heteroatoms selected from N and O on the ring; optionally, the heterocyclic ring is a 3- to 6-membered non-aromatic ring containing 1 or 2 heteroatoms selected from N and O on the ring; optionally, the heterocyclic ring is a 3- to 10-membered non-aromatic ring containing 1 or 2 heteroatoms selected from N and S on the ring; and optionally, the heterocyclic ring is a 3- to 6-membered non-aromatic ring containing 1 or 2 heteroatoms selected from N and S on the ring;
optionally, the "aryl" is 6- to 10-membered aryl, optionally phenyl or naphthyl, and optionally phenyl, 1-naphthyl, or 2-naphthyl;
optionally, the "arylene" is 6- to 10-membered arylene, and optionally phenylene or naphthylene;
optionally, the "heteroaryl" is a 5- to 10-membered heteroaromatic ring containing 1 to 3 heteroatoms selected from N, O, and S on the ring, and optionally a 5- to 10-membered heteroaromatic ring containing 1 to 2 heteroatoms selected from N, O, and S on the ring; optionally, the heteroaromatic ring is selected from a pyridine ring, a pyrrole ring, a pyrimidine ring, a pyrazine ring, a pyridazine ring, a thiophene ring, and a furan ring; optionally, the heteroaryl is selected from pyridin-2-yl, pyridin-3-yl, pyridin-4-yl, pyridazin-3-yl, pyridazin-4-yl, pyrimidin-2-yl, pyrimidin-4-yl, pyrimidin-5-yl, pyrazin-2-yl, pyrazin-3-yl, indolyl, isoindolyl, indazolyl, indolizinyl, purinyl, quinolizinyl, quinolinyl, isoquinolinyl, cinolinyl , phthalazinyl, naphthyridinyl, quinazolinyl, quinoxalinyl, thieno[2,3-b]furyl, furo[3,2-b]-pyranyl, pyrido[2,3-d]oxazinyl, pyrazolo[4,3-d]oxazolyl, imidazo[4,5-d]thiazolyl, pyrazino[2,3-d]pyridazinyl, imidazo[2,1-b]thiazolyl, imidazo[1,2-b][1,2,4]triazinyl, benzothienyl, benzoxazolyl, benzimidazolyl, benzothiazolyl, benzoxepinyl, benzoxazinyl, benzofuranyl, benzotriazolyl, pyrrolo[2,3-b]pyridyl, pyrrolo[3,2-c]pyridyl, pyrrolo[3,2-b]pyridyl, imidazo[4,5-b]pyridyl, imidazo[4,5-c]pyridyl, pyrazolo[4,3-d]pyridyl, pyrazolo[4,3-c]pyridyl, pyrazolo[3,4-c]pyridyl, pyrazolo[3,4-d]pyridyl, pyrazolo[3,4-b]pyridyl, imidazo[1,2-a]pyridyl, pyrazolo[1,5-a]pyridyl, pyrrolo[1,2-b]pyridazinyl, imidazo[1,2-c]pyrimidinyl, pyrido[3,2-d]pyrimidinyl, pyrido[4,3-d]pyrimidinyl, pyrido[3,4-d]pyrimidinyl, pyrido[2,3-d]pyrimidinyl, pyrido[2,3-b]pyrazinyl, pyrido[3,4-b]pyrazinyl, pyrimido[5,4-d]pyrimidinyl, pyrazolo[2,3-b]pyrazinyl, and pyrimido[4,5-d]pyrimidinyl, and optionally pyridin-2-yl, pyridin-3-yl, pyridin-4-yl, pyrimidin-2-yl, pyrimidin-4-yl, or pyrimidin-5-yl;
optionally, the "heteroarylene" is a 5- to 10-membered heteroarylene ring containing 1 to 3 heteroatoms selected from N, O, and S on the ring, and optionally a 5- to 10-membered heteroarylene ring containing 1 to 2 heteroatoms selected from N, O, and S on the ring; and optionally, the heteroarylene ring is selected from a pyridylidene ring, a pyrrylidene ring, a pyrimidylidene ring, a pyrazinylidene ring, a pyridazinylidene ring, a thienylidene ring, a furylidene ring.
optionally, n₁, n₂, n₃, and n₄ are each independently 0, 1, or 2; optionally, n₁ is 0, optionally, n₂ is 0 or 1, optionally, n₃ is 0, optionally, n₄ is 1;
optionally, R₁ and R₂ are each independently selected from: -H, F, Cl, Br, hydroxyl, cyano, C₁-C₇ alkyl (methyl, ethyl, n-propyl, isopropyl, n-butyl, isobutyl, tert-butyl, sec-butyl, n-pentyl, 1-methylbutyl, 2-methylbutyl, 3-methylbutyl, isopentyl, 1-ethylpropyl, neopentyl, n-hexyl, 1-methylpentyl, 2-methylpentyl, 3-methylpentyl, isohexyl, 1,1-dimethylbutyl, 2,2-dimethylbutyl, 3,3-dimethylbutyl, 1,2-dimethylbutyl, 1,3-dimethylbutyl, 2,3-dimethylbutyl, 2-ethylbutyl, n-heptyl, 2-methylhexyl, 3-methylhexyl, 2,2-dimethylpentyl, 3,3-dimethylpentyl, 2,3-dimethylpentyl, 2,4-dimethylpentyl, 3-ethylpentyl, or 2,2,3-trimethylbutyl), C₁-C₃ deuteroalkyl (-CD₃, -C₂D₅, -C₃D₇), cyclopropanyl, cyclobutanyl, and cyclopentyl; optionally, R₁ is -H, optionally, R₂ is -H;
optionally, X₁, X₂, and X₃ are each independently selected from: C₁-C₇ alkylene (optionally, -CH₂-, ethylene, n-propylidene, isopropylidene, n-butylidene, or isobutylidene), -O-, -S-, or - NR'-; optionally, X₁ is -CH₂-; optionally, X₂ is selected from -CH₂- or -O-; optionally, X₃ is -O-;
optionally, R' is selected from-H, C₁-C₇ alkyl (methyl, ethyl, n-propyl, isopropyl, n-butyl, isobutyl, tert-butyl, sec-butyl, n-pentyl, 1-methylbutyl, 2-methylbutyl, 3-methylbutyl, isopentyl, 1-ethylpropyl, neopentyl, n-hexyl, 1-methylpentyl, 2-methylpentyl, 3-methylpentyl, isohexyl, 1,1-dimethylbutyl, 2,2-dimethylbutyl, 3,3-dimethylbutyl, 1,2-dimethylbutyl, 1,3-dimethylbutyl, 2,3-dimethylbutyl, 2-ethylbutyl, n-heptyl, 2-methylhexyl, 3-methylhexyl, 2,2-dimethylpentyl, 3,3-dimethylpentyl, 2,3-dimethylpentyl, 2,4-dimethylpentyl, 3-ethylpentyl, or 2,2,3-trimethylbutyl;), deuteroalkyl (optionally -CD₃, -C₂D₅, -C₃D₇), or C₃-C₆ cycloalkyl (cyclopropanyl, cyclobutanyl, cyclopentyl, or cyclohexyl);
optionally, Ar is phenylene or pyridyl, and a hydrogen atom in the phenylene or pyridyl is optionally substituted by 1, 2, or 3 substituents that are each independently selected from: F, Cl, Br, I, -CN, -Me, -C₂H₅, cyclopropyl, -CD₃, -OMe, or -OCF₃; optionally, Ar is phenylene, and a hydrogen atom in the phenylene is optionally substituted by 2 substituents that are F;
optionally, Y is -H, -F, -Cl, -Br, methyl, ethyl, n-propyl, isoproyl, -CD₃, -CF₃, -CH₂CF₃, - OCF₃, -OCHF₂, cyclopropyl, -cyclobutyl, -cyclopentyl, -OCH3, -OC₂H₅, -OC₃H₇, or -OAr'; optionally, Y is -H or -OAr';
optionally, Ar' is selected from phenyl, pyridyl, pyrimidinyl, or quinolinyl, and hydrogen atoms in the phenyl, pyridyl, pyrimidinyl or quinolinyl ring are each independently optionally substituted with 1, 2 or 3 substituents that are each independently selected from: F, Cl, Br, -CN, C₁-C₇ alkyl (optionally selected from methyl, ethyl, n-propyl, isopropyl, n-butyl, isobutyl, tert-butyl, sec-butyl, n-pentyl, 1-methylbutyl, 2-methylbutyl, 3-methylbutyl, isopentyl, 1-ethylpropyl, neopentyl, n-hexyl, 1-methylpentyl, 2-methylpentyl, 3-methylpentyl, isohexyl, 1,1-dimethylbutyl, 2,2-dimethylbutyl, 3,3-dimethylbutyl, 1,2-dimethylbutyl, 1,3-dimethylbutyl, 2,3-dimethylbutyl, 2-ethylbutyl, n-heptyl, 2-methylhexyl, 3-methylhexyl, 2,2-dimethylpentyl, 3,3-dimethylpentyl, 2,3-dimethylpentyl, 2,4-dimethylpentyl, 3-ethylpentyl, or 2,2,3-trimethylbutyl), -CD₃, C₁-C₆haloalkyl, -OCH₃, -OC₂H₇, -OC₃H₇, C₁-C₆ haloalkoxyl, or C₃-C₆cycloalkyl (optionally cyclopropanyl, cyclobutanyl, cyclopentyl, or cyclohexyl); optionally, Ar' is selected from phenyl, pyridin-3-yl, or pyridin-4-yl, or pyrimidin-5-yl, optionally, the Ar' is substituted by 1 or 2 substituents that are selected from Cl, -CH₃, -CF₃, or -OCF₃; and optionally, Z is O.

Optionally, the compound of formula (I), or the pharmaceutically acceptable salt thereof, wherein the compound of formula (I) is selected from the following compounds:

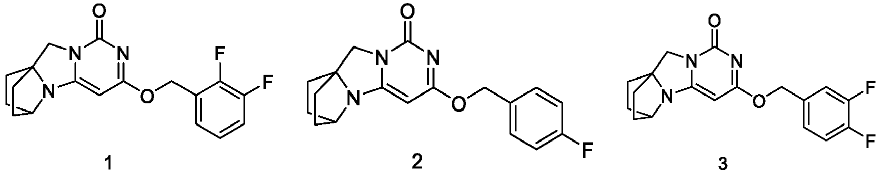

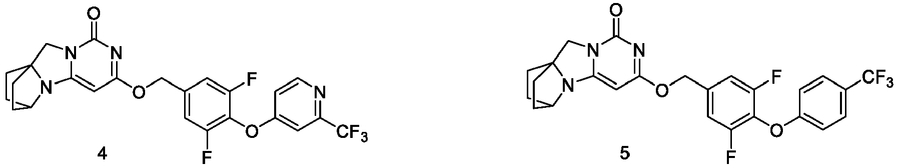

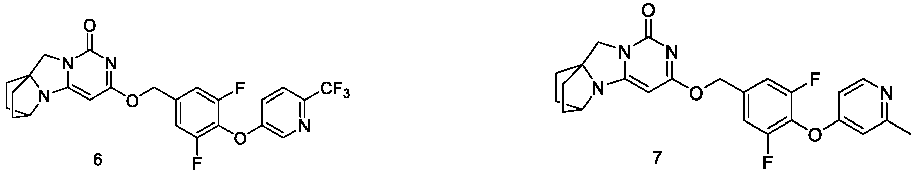

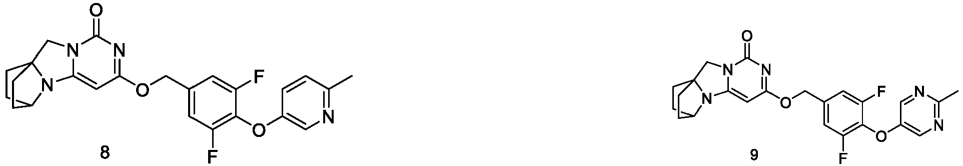

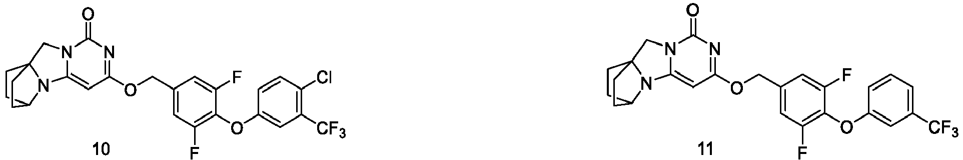

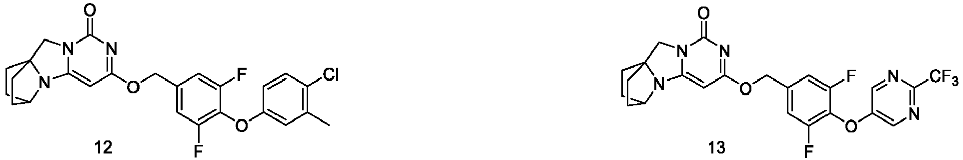

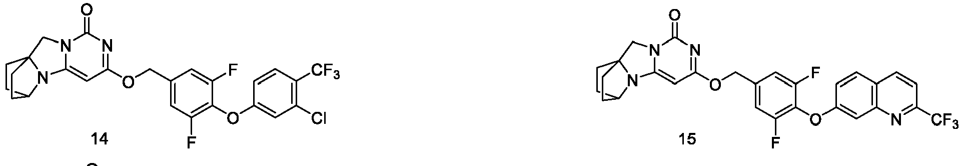

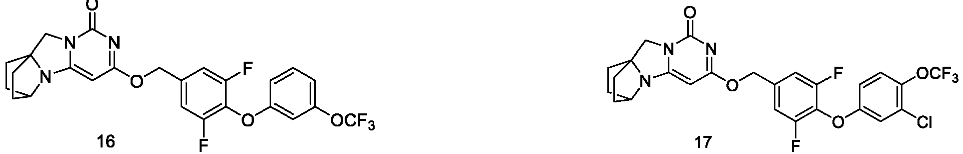

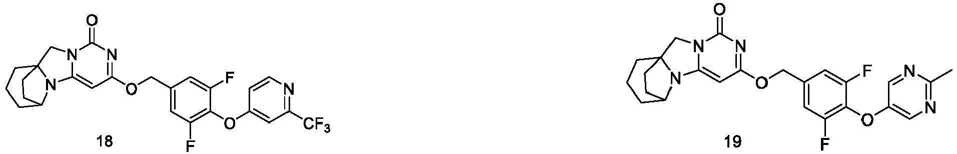

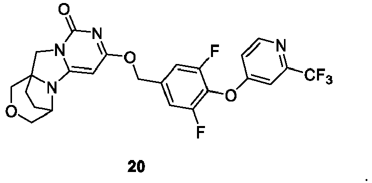

Optionally, the compound of formula (I), or the pharmaceutically acceptable salt thereof, wherein the pharmaceutically acceptable salt includes an anionic salt and a cationic salt of the compound of formula (I);
optionally, the pharmaceutically acceptable salt includes an alkali metal salt, an alkaline earth metal salt, and an ammonium salt of the compound of formula (I); optionally, the alkali metal includes sodium, potassium, lithium, and cesium, and the alkaline earth metal includes magnesium, calcium, and strontium;
optionally, the pharmaceutically acceptable salt includes a salt formed by the compound of formula (I) and an organic base;
optionally, the organic base includes trialkylamine, pyridine, quinoline, piperidine, imidazole, picoline, dimethylaminopyridine, dimethylaniline, N-alkylmorpholine, 1,5-diazabicyclo[4.3.0]nonene-5, 1,8-diazabicyclo[5.4.0]undecene-7, and 1,4-diazabicyclo[2.2.2]octane; optionally, the trialkylamine includes trimethylamine, triethylamine, and N-ethyldiisopropylamine; and optionally, the N-alkylmorpholine includes N-methylmorpholine;
optionally, the pharmaceutically acceptable salt includes a salt formed by the compound of formula (I) and an acid; and
optionally, the acid includes an inorganic acid and an organic acid; optionally, the inorganic acid includes hydrochloric acid, hydrobromic acid, hydroiodic acid, sulfuric acid, nitric acid, phosphoric acid, carbonic acid; optionally, the organic acid includes formic acid, acetic acid, propionic acid, oxalic acid, malonic acid, succinic acid, fumaric acid, maleic acid, lactic acid, malic acid, citric acid, citric acid, tartaric acid, carbonic acid, picric acid, methanesulfonic acid, ethanesulfonic acid, *p*-toluenesulfonic acid, glutamic acid, and pamoic acid.

In another aspect, provided is a method for preparing a compound of formula (I), or a pharmaceutically acceptable salt thereof, the method comprising the following reaction route: 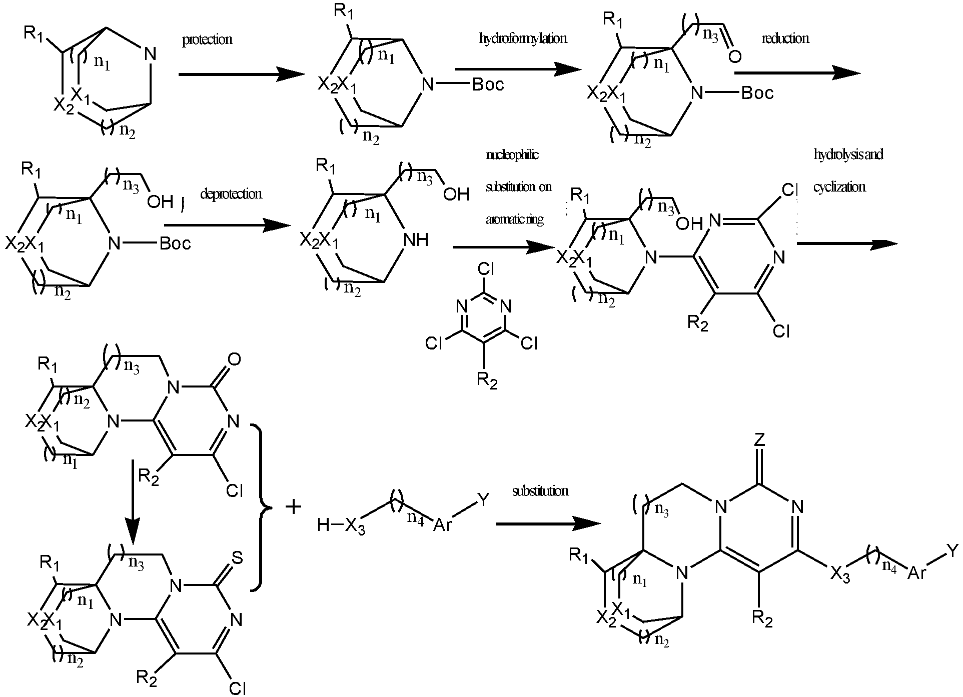
in respective formulas, n₁, n₂, n₃, R₁, R₂, X₁, X₂, X₃, Z, Ar, and Y are as defined above;
optionally, when X₂ is -O- and n₃ is 0, the following synthetic route is further adopted:

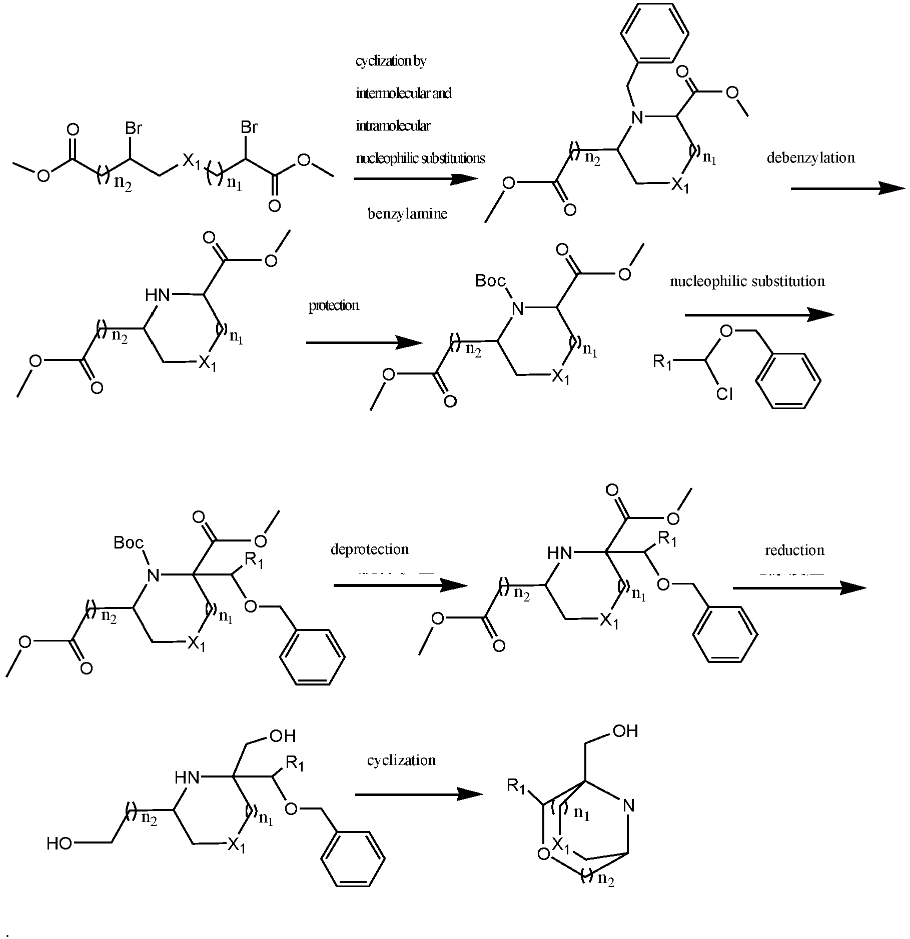

The specific reaction conditions for each of the above-mentioned reactions are not particularly limited, and existing conventional reaction conditions or steps can be used.

In another aspect, provided is a pharmaceutical composition, comprising a therapeutically effective dose of one or more of the above-mentioned compound of formula (I), or pharmaceutically acceptable salt thereof, and optionally a pharmaceutically acceptable carrier.
optionally, a dosage form of the pharmaceutical composition includes an oral preparation, a rectally administered preparation, and a parenteral administered preparation;
optionally, the oral preparation includes a solid preparation and a liquid preparation;
optionally, the solid preparation includes tablets, powders, granules, and capsules;
optionally, the liquid preparation includes aqueous or oily suspensions, and syrups; and
optionally, the parenteral administered preparation includes solutions for injection, and aqueous or oily suspensions.

In another aspect, provided is use of a compound of formula (I) or a pharmaceutically acceptable salt thereof as described above, or a pharmaceutical composition as described above in the preparation of an Lp-PLA2 inhibitor.

In another aspect, provided is use of a compound of formula (I) or a pharmaceutically acceptable salt thereof as described above, or a pharmaceutical composition as described above in the preparation of a medicament for treating a neurodegenerative-related disease; and
optionally, the neurodegenerative-related disease includes Alzheimer's disease (AD), glaucoma, and age-related macular degeneration (AMD).

In another aspect, provided is use of a compound of formula (I) or a pharmaceutically acceptable salt thereof as described above, or a pharmaceutical composition as described above in the preparation of a medicament for treating cardiovascular disease, diabetic macular edema (DME), or prostatic disease; and
optionally, the cardiovascular disease includes atherosclerosis.

### Beneficial Effects:

The compound of formula (I) is a tetracyclic pyrimidinone compound, which is a brand new Lp-PLA2 inhibitor. It is useful for treating neurodegenerative-related diseases, such as Alzheimer's disease (AD), glaucoma and age-related macular degeneration (AMD), or cardiovascular diseases including atherosclerosis.

### DETAILED DESCRIPTION

The present disclosure will be further explained with reference to the following examples. It should be appreciated that the examples described herein are merely intended to exemplarily illustrate the present disclosure, and not limit the scope of the present disclosure in any way.

The starting materials of the present disclosure can be synthesized by or according to the methods known in the art, or can be purchased from ABCR GmbH & Co. KG, Acros Organics, Aldrich Chemical Company, Accela ChemBio Inc., Darui Chemicals, and other companies.

Unless otherwise specified in the examples, the solution refers to an aqueous solution.

Unless otherwise specified in the examples, the reaction temperature is room temperature, e.g., 20°C to 30°C.

### Example 1

### Preparation of Compound 1

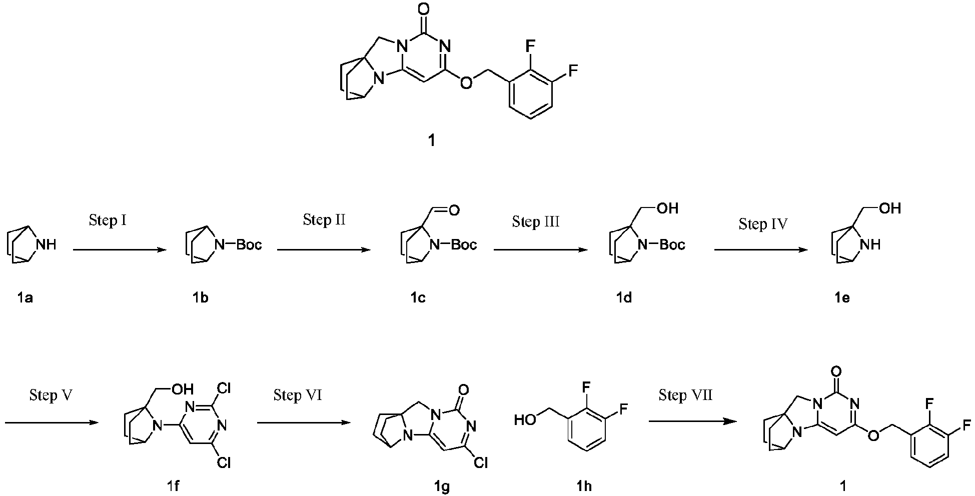

### Step I: Preparation of Compound 1b

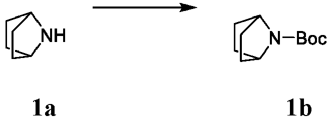

At room temperature, 7-azabicyclo[2.2.1]heptane **1a** (4 g, 41.1 mmol) was dissolved in 300 mL of dichloromethane, followed by addition of triethylamine (6.3 g, 61.8 mmol) and di-tert-butyl dicarbonate (13.5 g, 61.8 mmol). The materials were stirred at room temperature and reacted for 4 h, and the reactant was extracted with dichloromethane (100 mL×3). The organic phases were combined, washed with saturated aqueous sodium chloride solution, dried over anhydrous sodium sulfate, and filtered to remove the drying agent. The filtrate was concentrated under reduced pressure, and purified with an eluent system (petroleum ether/ethyl acetate = 20:1) by the silica gel column chromatography to give a colorless, oily product **1b** (10 g, yield: 100%). ¹H NMR (400 MHz, CDCl₃) δ 4.16 (s, 2H), 1.74 - 1.72 (m, 4H), 1.42 (s, 9H), 1.37-1.35 (m, 4H).

### Step II: Preparation of Compound 1c

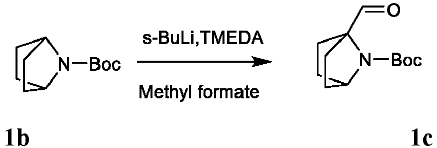

At room temperature, tert-butyl 7-azabicyclo[2.2.1]heptane-7-carboxylate **1b** (10 g, 50.8 mmol) and TMEDA (7.1 g, 60.9 mmol) were dissolved in 200 mL of dry ether, followed by adding dropwise a solution of s-BuLi (1.3 M, 60.9 mmol) in hexane at -65°C under nitrogen protection. After the materials were stirred and reacted for 30 min, a solution of methyl formate (3.65 g, 60.9 mmol) in ether was added dropwise. This mixture was stirred and reacted at -65°C for 30 min, heated to 0°C, and reacted for 2 h. To the reaction mixture, 100 mL of saturated aqueous ammonium chloride solution was added, and extracted with ether (50 mL×3). The organic phases were combined, dried over anhydrous sodium sulfate, and filtered to remove the drying agent. The filtrate was concentrated under reduced pressure, and purified with an eluent system (petroleum ether/ethyl acetate = 40:1) by the silica gel column chromatography to give a colorless, oily product **1c** (8.14 g, yield: 71%). ¹H NMR (400 MHz, CDCl₃) δ 9.93 (s, 1H), 4.29 (s, 1H), 2.03 - 1.88 (m, 4H), 1.65 - 1.46 (m, 4H), 1.42 (s, 9H).

### Step III: Preparation of Compound 1d

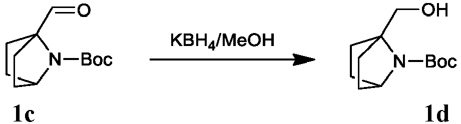

Tert-butyl 1-formyl-7-azabicyclo[2.2.1]heptane-7-carboxylate **1c** (8.14 g, 36.1 mmol) was dissolved in 300 mL of methanol, to which KBH₄ (3.03 g, 54.2 mmol) was added in batch at 0°C. After the materials were stirred and reacted at room temperature for 1 h, the reactant was concentrated under reduced pressure, and extracted with dichloromethane (60 mL×3). The organic phases were combined, washed with saturated aqueous sodium chloride solution, dried over anhydrous sodium sulfate, and filtered to remove the drying agent. The filtrate was concentrated under reduced pressure, and purified with an eluent system (petroleum ether/ethyl acetate = 20/1) by the silica gel column chromatography to give a colorless, oily product **1d** (6.53 g, yield: 79.5%). ¹H NMR (400 MHz, CDCl₃) δ4.96 (s, 1H), 4.24 (m, 1H), 3.90 (m, 2H), 1.89 - 1.77 (m, 4H), 1.48 - 1.35 (m, 13H).

### Step IV: Preparation of Compound 1e

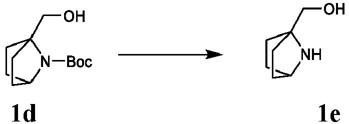

Tert-butyl 1-(hydroxymethyl)-7-azabicyclo[2.2.1]heptane-7-carboxylate **1d** (6.53 g, 28.7 mmol) was dissolved in 100 mL of hydrochloric acid/ethanol (2M). After the materials were stirred and reacted at room temperature for 4 h, the reactant was concentrated under reduced pressure to give a yellow solid product **1e** (4.72 g, yield: 100%).

¹H NMR (400 MHz, DMSO) δ 9.04 (s, 2H), 3.98 (m, 1H), 3.73 (s, 2H), 1.98-1.82 (m, 4H), 1.69-1.53 (m, 4H).

### Step V: Preparation of Compound 1f

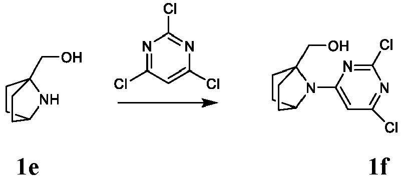

At room temperature, (7-azabicyclo[2.2.1]heptane-1-yl)methanol **1e** (4.5 g, 35.5 mmol), 2,4,6-trichloropyrimidine (7.79 g, 42.6 mmol), and diisopropylethylamine (13.7 g, 106.5 mmol) were dissolved in 200 mL of acetonitrile, stirred overnight, concentrated under reduced pressure, and purified with an eluent system (petroleum ether/ethyl acetate = 5:1) by the silica gel column chromatography to give a colorless solid product **1f** (5.2 g, yield: 53%).

¹H NMR (400 MHz, CDCl₃) δ 6.37 (s, 1H), 4.91 (m, 1H), 4.26 (m, 1H), 4.01 (m, 2H), 2.01 - 1.82 (m, 4H), 1.68-1.52 (m, 4H).

### Step VI: Preparation of Compound 1g

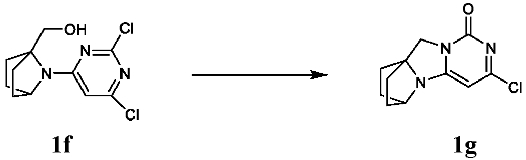

At room temperature, (7-(2,6-dichloropyrimidin-4-yl)-7-azabicyclo[2.2.1]heptan-1-yl)methanol **1f** (5.46 g, 19.9 mmol) and triethylamine (6.0 g, 59.7 mmol) were dissolved in 100 mL of dry dichloromethane, to which MsCl (2.5 g, 21.9 mmol) was added dropwise at 0°C. After the materials were stirred and reacted at 0°C for 1 h, the reactant was concentrated under reduced pressure, and dissolved in 100 mL of a mixed solvent of dioxane/water (1:1), to which potassium carbonate (8.2 g, 59.7 mmol) was added, and stirred at 90°C overnight. Then the reactant was concentrated under reduced pressure, and purified with an eluent system (dichloromethane/methanol = 20:1) by the silica gel column chromatography to give a colorless solid product **1g** (2.2 g, yield: 46%).

¹H NMR (400 MHz, DMSO) δ 6.10 (s, 1H), 4.42 (m, 1H), 3.95 (s, 2H), 1.94 -1.89 (m, 2H), 1.78 - 1.69 (m, 6H).

### Step VII: Preparation of Compound 1

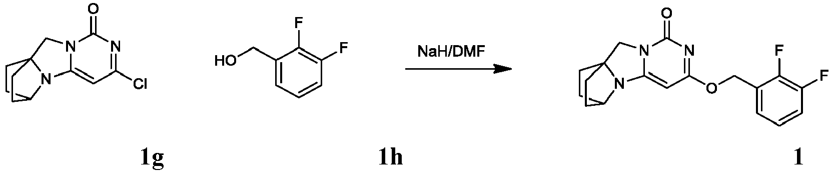

(2,3-Difluorophenyl)methanol **1h** (46 mg, 0.32 mmol) was dissolved in 2 mL of dry *N,N-*dimethylformamide for 1 h, to which sodium hydride (60% in mineral oil, 17 mg, 0.42 mmol) was added at 0°C. After the materials were stirred and reacted at room temperature for 20 min, Compound **1g** (50 mg, 0.21 mmol) was added, and stirred and reacted for 1 h. Thereafter, a small amount of water was added to quench the reaction. The reactant was purified with an eluent system (dichloromethane/methanol = 20:1) by the silica gel column chromatography to give a white solid product 1 (31 mg, yield: 43%).

¹H NMR (400 MHz, CDCl₃) δ 7.24 - 7.03 (m, 3H), 5.45 (s, 2H), 5.15 (s, 1H), 4.09 (m, 1H), 3.99 (s, 2H), 2.14 - 1.99 (m, 2H), 1.78 - 1.66 (m, 6H). MS (ESI): m/z 346.0 [M+H]⁺.

### Example 2 Preparation of Compound 2

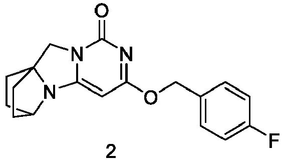

(4-Fluorophenyl)methanol (40 mg, 0.32 mmol) was dissolved in 5 mL of dry *N,N-*dimethylformamide, to which sodium hydride (60% in mineral oil, 17 mg, 0.42 mmol) was added at 0°C. After the materials were stirred and reacted at room temperature for 20 min, Compound **1g** (50 mg, 0.21 mmol) was added, and stirred and reacted for 1 h. Thereafter, a small amount of water was added to quench the reaction. The reactant was purified with an eluent system (dichloromethane/methanol = 20:1) by the silica gel column chromatography to give a white solid product **2** (40 mg, yield: 58%).

¹H NMR (400 MHz, CDCl₃) δ 7.43 - 7.40 (m, 2H), 7.08 - 7.04 (m, 2H), 5.37 (s, 2H), 5.17 (s, 1H), 4.11 (m, 1H), 4.01 (s, 2H), 2.10 - 2.06 (m, 2H), 1.82 - 1.67 (m, 6H). MS (ESI): m/z 328.0 [M+H]⁺.

### Example 3 Preparation of Compound 3

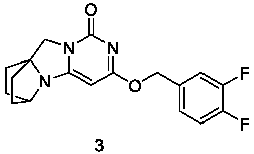

(3,4-Dichlorophenyl)methanol (46 mg, 0.32 mmol) was dissolved in 5 mL of dry *N,N-*dimethylformamide, to which sodium hydride (60% in mineral oil, 17 mg, 0.42 mmol) was added at 0°C. After the materials were stirred and reacted at room temperature for 20min, Compound **1g** (50 mg, 0.21 mmol) was added, and stirred and reacted for 1 h. Thereafter, a small amount of water was added to quench the reaction. The reactant was purified with an eluent system (dichloromethane/methanol = 20:1) by the silica gel column chromatography to give a white solid product **3** (17 mg, yield: 23%).

¹H NMR (400 MHz, CDCl₃) δ 7.28 - 7.22 (m, 1H), 7.18 - 7.11 (m, 2H), 5.35 (s, 2H), 5.17 (s, 1H), 4.12 (m, 1H), 4.01 (s, 2H), 2.12 - 2.07 (m, 2H), 1.83 - 1.74 (m, 6H). MS (ESI): m/z 346.0 [M+H]⁺.

### Example 4 Preparation of Compound 4

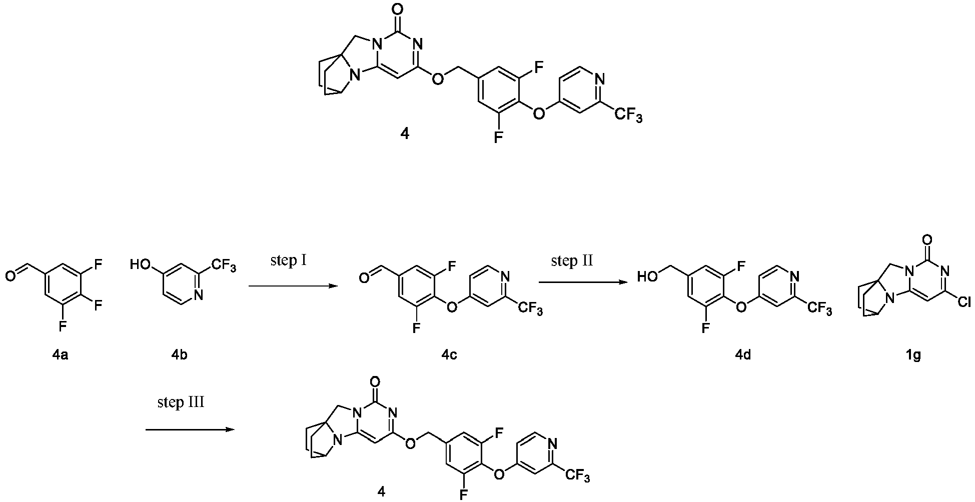

### Step I: Preparation of Compound 4c

At room temperature, 2-(trifluoromethyl)pyridin-4-ol **4b** (0.85 g, 5.2 mmol), 3,4,5-trifluorobenzaldehyde (1 g, 6.2 mmol) and potassium carbonate (0.93 g, 6.76 mmol) were dissolved in 30 mL of *N,N*-dimethylformamide, and stirred and reacted at 90°C for 1 h. The reactant was cooled to room temperature, then poured into 100 mL of ice water, and extracted with ethyl acetate (50 mL×3). The organic phases were combined, washed with saturated aqueous sodium chloride solution, dried over anhydrous sodium sulfate, and filtered to remove the drying agent. The filtrate was concentrated under reduced pressure, and purified with an eluent system (petroleum ether/ethyl acetate = 5/1) by the silica gel column chromatography to give a yellow solid product **4c** (1.47g, yield: 78.2%).

¹H NMR (400 MHz, CDCl₃) δ 9.97 (s, 1H), 8.65 (m, 1H), 7.63 (m, 2H), 7.27 (m, 1H), 7.01 (m, 1H).

### Step II: Preparation of Compound 4d

At room temperature, 3,5-difluoro-4-((2-(trifluoromethyl)pyridin-4-yl)oxy)benzaldehyde **4c** (1.47 g, 4.85 mmol) was dissolved in 50 mL of ethanol, to which NaBH₄ (184 mg, 4.85 mmol) was added at 0°C. The materials were stirred and reacted at room temperature for 0.5 h, concentrated under reduced pressure, added with water, and extracted with ethyl acetate (100 mL×2). The organic phases were combined, washed with saturated aqueous sodium chloride solution, dried over anhydrous sodium sulfate, and filtered to remove the drying agent. The filtrate was concentrated under reduced pressure, and purified with an eluent system (petroleum ether/ethyl acetate = 2:1) by the silica gel column chromatography to give a white solid product **4d** (1.04 g, yield: 70.3%).

¹H NMR (400 MHz, CDCl₃) δ 8.59 (m, 1H), 7.24 (m, 1H), 7.11 (m, 2H), 6.99 (m, 1H), 4.75 (m, 2H), 2.19 (m, 1H).

### Step III: Preparation of Compound 4

(3,5-Difluoro-4-((2-(trifluoromethyl)pyridin-4-yl)oxy)phenyl)methanol (77 mg, 0.25 mmol) was dissolved in 5 mL of dry *N,N*-dimethylformamide, to which sodium hydride (60% in mineral oil, 17 mg, 0.42 mmol) was added at 0°C. After the materials were stirred and reacted at room temperature for 5 min, Compound **1g** (50 mg, 0.21 mmol) was added, and stirred and reacted for 1 h. Thereafter, a small amount of water was added to quench the reaction. The reactant was purified with an eluent system (dichloromethane/methanol = 20:1) by the silica gel column chromatography to give a white solid product 4 (35 mg, yield: 33%).

¹H NMR (400 MHz, CDCl₃) δ 8.62 (m, 1H), 7.28 (s, 1H), 7.15 (m, 2H), 7.01 (m, 1H), 5.44 (s, 2H), 5.24 (s, 1H), 4.16 (m, 1H), 4.03 (s, 2H), 2.12 - 2.07 (m, 2H), 1.83 - 1.79 (m, 6H). MS (ESI): m/z 506.9 [M+H]⁺.

### Example 5 Preparation of Compound 5

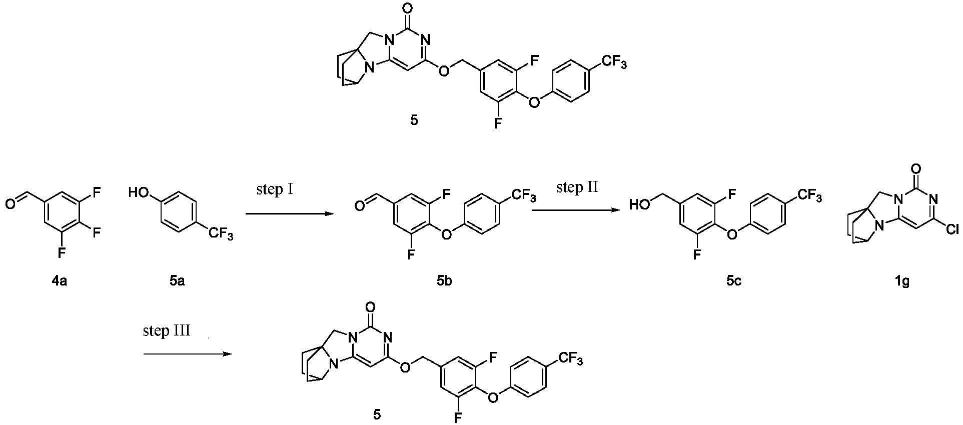

### Step I: Preparation of Compound 5b

At room temperature, 4-(trifluoromethyl)phenol **5a** (0.84 g, 5.2 mmol), 3,4,5-trifluorobenzaldehyde **4a** (1 g, 6.2 mmol) and potassium carbonate (0.93 g, 6.76 mmol) were dissolved in 30 mL *N,N*-dimethylformamide, and stirred and reacted at 90°C for 1 h. The reactant was cooled to room temperature, then poured into 100 mL of ice water, and extracted with ethyl acetate (50 mL×3). The organic phases were combined, washed with saturated aqueous sodium chloride solution, dried over anhydrous sodium sulfate, and filtered to remove the drying agent. The filtrate was concentrated under reduced pressure, and purified with an eluent system (petroleum ether/ethyl acetate = 5/1) by the silica gel column chromatography to give a yellow solid product **5b** (1.33 g, yield: 71.0%).

¹H NMR (400 MHz, CDCl₃) δ 9.94 (m, 1H), 7.59 (m, 4H), 7.04 (m, 2H).

### Step II: Preparation of Compound 5c

At room temperature, 3,5-difluoro-4-(4-(trifluoromethyl)phenoxy)benzaldehyde **5b** (1.33 g, 4.4 mmol) was dissolved in 50 mL of methylethanol, to which NaBH₄ (166 mg, 4.4 mmol) was added at 0°C. The materials were stirred and reacted at room temperature for 0.5 h, concentrated under reduced pressure, added with water, and extracted with ethyl acetate (100 mL×2). The organic phases were combined, washed with saturated aqueous sodium chloride solution, dried over anhydrous sodium sulfate, and filtered to remove the drying agent. The filtrate was concentrated under reduced pressure, and purified with an eluent system (petroleum ether/ethyl acetate = 2/1) by the silica gel column chromatography to give a colorless, oily product **5c** (0.85g, yield: 63.6 %).

¹H NMR (400 MHz, CDCl₃) δ 7.57 (m, 2H), 7.09 - 7.00 (m, 4H), 4.72 (m, 2H), 2.03 (m, 1H).

### Step III: Preparation of Compound 5

3,5-Difluoro-4-(4-(trifluoromethyl)phenoxy)phenyl)methanol **5c** (64 mg, 0.21 mmol) was dissolved in 5 mL of dry *N,N*-dimethylformamide, to which sodium hydride (60% in mineral oil, 17 mg, 0.42 mmol) was added at 0°C. After the materials were stirred and reacted at room temperature for 20 min, Compound **1g** (50 mg, 0.21 mmol) was added, and stirred and reacted for 1 h. Thereafter, a small amount of water was added to quench the reaction. The reactant was purified with an eluent system (dichloromethane/methanol = 20:1) by the silica gel column chromatography to give a white solid product **5** (17 mg, yield: 16%).

¹H NMR (400 MHz, CDCl₃) δ 7.59 (m, 2H), 7.12 - 7.02 (m, 4H), 5.42 (s, 2H), 5.23 (s, 1H), 4.15 (m, 1H), 4.03 (s, 2H), 2.12 (m, 2H), 1.87 - 1.74 (m, 6H). MS (ESI): m/z 506.0 [M+H]⁺.

### Example 6 Preparation of Compound 6

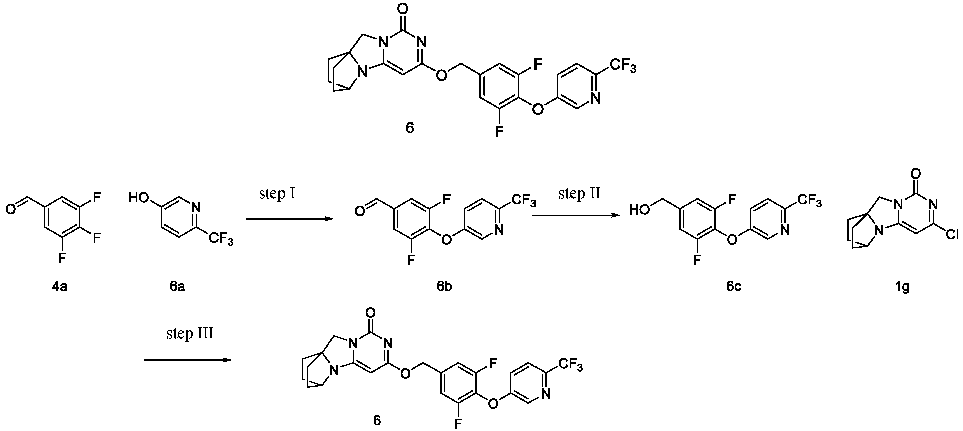

### Step I: Preparation of Compound 6b

At room temperature, 6-(trifluoromethyl)pyridin-3-ol **6a** (0.85 g, 5.2 mmol), 3,4,5-trifluorobenzaldehyde **4a** (1 g, 6.2 mmol) and potassium carbonate (0.93 g, 6.76 mmol) were dissolved in 30 mL of *N,N*-dimethylformamide, and stirred and reacted at 90°C for 1 h. The reactant was cooled to room temperature, then poured into 100 mL of ice water, and extracted with ethyl acetate (50 mL×3). The organic phases were combined, washed with saturated aqueous sodium chloride solution, dried over anhydrous sodium sulfate, and filtered to remove the drying agent. The filtrate was concentrated under reduced pressure, and purified with an eluent system (petroleum ether/ethyl acetate = 5/1) by the silica gel column chromatography to give a yellow solid product **6b** (1.34 g, yield: 84.6 %).

### Step II: Preparation of Compound 6c

At room temperature, 3,5-difluoro-4-((6-(trifluoromethyl)pyridin-3-yl)oxy)benzaldehyde **6b** (1.34 g, 4.4 mmol) was dissolved in 50 mL of methanol, to which NaBH₄ (167 mg, 4.4 mmol) was added at 0°C, stirred and reacted at room temperature for 0.5 h, concentrated under reduced pressure, added with water, and extracted with ethyl acetate (100 mL×2). The organic phases were combined, washed with saturated aqueous sodium chloride solution, dried over anhydrous sodium sulfate, and filtered to remove the drying agent. The filtrate was concentrated under reduced pressure, and purified with an eluent system (petroleum ether/ethyl acetate = 2:1) by the silica gel column chromatography to give a colorless, oily product **6c** (0.77 g, yield: 57.4 %).

¹H NMR (400 MHz, CDCl₃) δ 8.46 (s, 1H), 7.63 (m, 1H), 7.30 (m, 1H), 7.09 (m, 2H), 4.73 (m, 2H), 2.40 (m, 1H).

### Step III: Preparation of Compound 6

(3,5-Difluoro-4-((6-(trifluoromethyl)pyridin-3-yl)oxy)phenyl)methanol **6c** (50 mg, 0.21 mmol) was dissolved in 5 mL of dry *N,N*-dimethylformamide, to which sodium hydride (60% in mineral oil, 17 mg, 0.42 mmol) was added at 0°C. After the materials were stirred and reacted at room temperature for 20 min, Compound **1g** (50 mg, 0.21 mmol) was added, and stirred and reacted for 1 h. Thereafter, a small amount of water was added to quench the reaction. The reactant was purified with an eluent system (dichloromethane/methanol = 20:1) by the silica gel column chromatography to give a white solid product 6 (31 mg, yield: 29%).

¹H NMR (400 MHz, CDCl₃) δ 8.51 (m, 1H), 7.65 (m, 1H), 7.31 (m, 1H), 7.13 (m, 2H), 5.42 (s, 2H), 5.23 (s, 1H), 4.15 (m, 1H), 4.03 (s, 2H), 2.12 - 2.06 (m, 2H), 1.85 - 1.70 (m, 6H). MS (ESI): m/z 507.0 [M+H]⁺.

### Example 7 Preparation of Compound 7

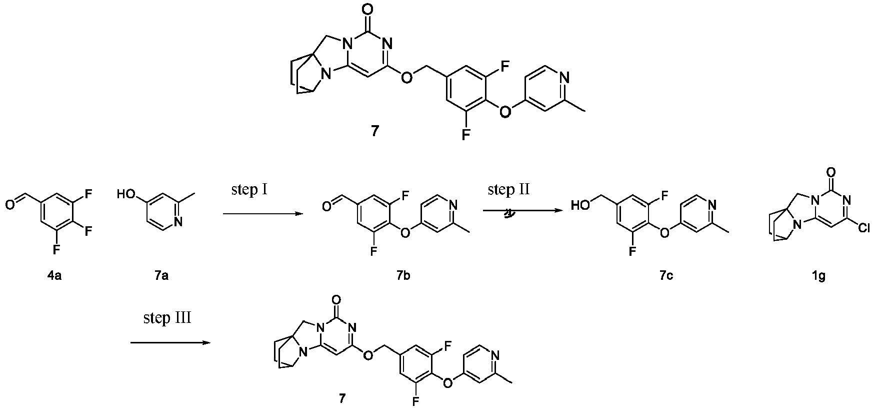

### Step I: Preparation of Compound 7b

At room temperature, 6-methylpyridin-4-ol **7a** (0.5 g, 4.6 mmol), 3,4,5-trifluorobenzaldehyde **4a** (0.88 g, 5.5 mmol) and potassium carbonate (0.823 g, 5.95 mmol) were dissolved in 30 mL of *N,N*-dimethylformamide, and stirred and reacted at 90°C for 2 h. The reactant was cooled to room temperature, then poured into 100 mL of ice water, and extracted with ethyl acetate (50 mL×3). The organic phases were combined, washed with saturated aqueous sodium chloride solution, dried over anhydrous sodium sulfate, and filtered to remove the drying agent. The filtrate was concentrated under reduced pressure, and purified with an eluent system (petroleum ether/ethyl acetate = 10/1) by the silica gel column chromatography to give a yellow solid product **7b** (0.4 g, yield: 33.8 %).

¹H NMR (400 MHz, CDCl₃) δ 9.94 (s, 1H), 8.39 (m, 1H), 7.62 - 7.56 (m, 2H), 6.70 - 6.66 (m, 2H), 2.52 (s, 3H).

### Step II: Preparation of Compound 7c

At room temperature, 3,5-difluoro-4-((2-methylpyridin-4-yl)oxy)benzaldehyde **7b** (0.4 g, 1.86 mmol) was dissolved in 50 mL of methanol, to which NaBH₄ (71 mg, 1.86 mmol) was added at 0°C, stirred and reacted at room temperature for 0.5 h, concentrated under reduced pressure, added with water, and extracted with ethyl acetate (100 mL×2). The organic phases were combined, washed with saturated aqueous sodium chloride solution, dried over anhydrous sodium sulfate, and filtered to remove the drying agent. The filtrate was concentrated under reduced pressure, and purified with an eluent system (petroleum ether/ethyl acetate = 4/1) by the silica gel column chromatography to give a colorless, oily product **7c** (0.40 g, yield: 85.7 %).

¹H NMR (400 MHz, CDCl₃) δ 8.29 (m, 1H), 7.07 (m, 2H), 6.70 - 6.64 (m, 2H), 4.73 (s, 2H), 3.20 (m, 1H), 2.50 (s, 3H).

### Step III: Preparation of Compound 7

(3,5-Difluoro-4-((2-methylpyridin-4-yl)oxy)phenyl)methanol **7c** (53 mg, 0.21 mmol) was dissolved in 5 mL of dry *N,N*-dimethylformamide, to which sodium hydride (60% in mineral oil, 17 mg, 0.42 mmol) was added at 0°C. After the materials were stirred and reacted at room temperature for 20 min, Compound **1g** (50 mg, 0.21 mmol) was added, and stirred and reacted for 1 h. Thereafter, a small amount of water was added to quench the reaction. The reactant was purified with an eluent system (dichloromethane/methanol = 20:1) by the silica gel column chromatography to give a white solid product 7 (17 mg, yield: 18%).

¹H NMR (400 MHz, CDCl₃) δ 8.38 (m, 1H), 7.10 (m, 2H), 6.69 (m, 2H), 5.42 (s, 2H), 5.23 (s, 1H), 4.15 (m, 1H), 4.03 (s, 2H), 2.53 (s, 3H), 2.21 - 2.07 (m, 2H), 1.88 - 1.70 (m, 6H). MS (ESI): m/z 453.0 [M+H]⁺.

### Example 8 Preparation of Compound 8

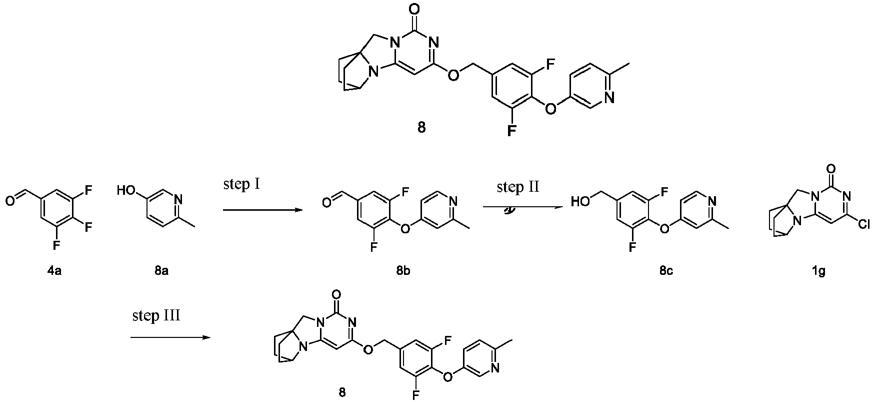

### Step I: Preparation of Compound 8b

At room temperature, 6-methylpyridin-3-ol **8a** (0.57 g, 5.2 mmol), 3,4,5-trifluorobenzaldehyde **4a** (1 g, 6.2 mmol) and potassium carbonate (0.93 g, 6.76 mmol) were dissolved in 30 mL of *N,N*-dimethylformamide, and stirred and reacted at 90°C for 1 h. The reactant was cooled to room temperature, then poured into 100 mL of ice water, and extracted with ethyl acetate (50 mL×3). The organic phases were combined, washed with saturated aqueous sodium chloride solution, dried over anhydrous sodium sulfate, and filtered to remove the drying agent. The filtrate was concentrated under reduced pressure, and purified with an eluent system (petroleum ether/ethyl acetate = 10/1) by the silica gel column chromatography to give a yellow solid product **8b** (0.91g, yield: 69.4 %).

¹H NMR (400 MHz, CDCl₃) δ 9.92 (s, 1H), 8.28 (s, 1H), 7.62 - 7.49 (m, 2H), 7.18 - 7.10 (m, 2H), 2.54 (s, 3H).

### Step II: Preparation of Compound 8c

At room temperature, 3,5-difluoro-4-((2-methylpyridin-4-yl)oxy)benzaldehyde **8b** (0.91 g, 4.3 mmol) was dissolved in 50 mL of methanol, to which NaBH₄ (161 mg, 4.3 mmol) was added at 0°C, stirred and reacted at room temperature for 0.5h, concentrated under reduced pressure, added with water, and extracted with ethyl acetate (100 mL×2). The organic phases were combined, washed with saturated aqueous sodium chloride solution, dried over anhydrous sodium sulfate, and filtered to remove the drying agent. The filtrate was concentrated under reduced pressure, and purified with an eluent system (petroleum ether/ethyl acetate = 4/1) by the silica gel column chromatography to give a colorless, oily product **8c** (0.89 g, yield: 82.5 %).

¹H NMR (400 MHz, CDCl₃) δ 8.20 (m, 1H), 7.16 - 6.98 (m, 4H), 4.69 (m, 2H), 2.88 (m, 1H), 2.50 (s, 3H).

### Step III: Preparation of Compound 8

(3,5-Difluoro-4-((2-methylpyridin-4-yl)oxy)phenyl)methanol **8c** (53 mg, 0.21 mmol) was dissolved in 2 mL of dry *N,N*-dimethylformamide, to which sodium hydride (60% in mineral oil, 17 mg, 0.42 mmol) was added at 0°C. After the materials were stirred and reacted at room temperature for 20 min, Compound **1g** (50 mg, 0.21 mmol) was added, and stirred and reacted for 1 h. Thereafter, a small amount of water was added to quench the reaction. The reactant was purified with an eluent system (dichloromethane/methanol = 20:1) by the silica gel column chromatography to give a white solid product **8** (20 mg, yield: 21%).

¹H NMR (400 MHz, CDCl₃) δ 8.30 (m, 1H), 7.18 - 7.04 (m, 4H), 5.40 (s, 2H), 5.22 (s, 1H), 4.15 (m, 1H), 4.03 (s, 2H), 2.54 (s, 3H), 2.11 (m, 2H), 1.86 - 1.74 (m, 6H). MS (ESI): m/z 453.0 [M+H]⁺.

### Example 9 Preparation of Compound 9

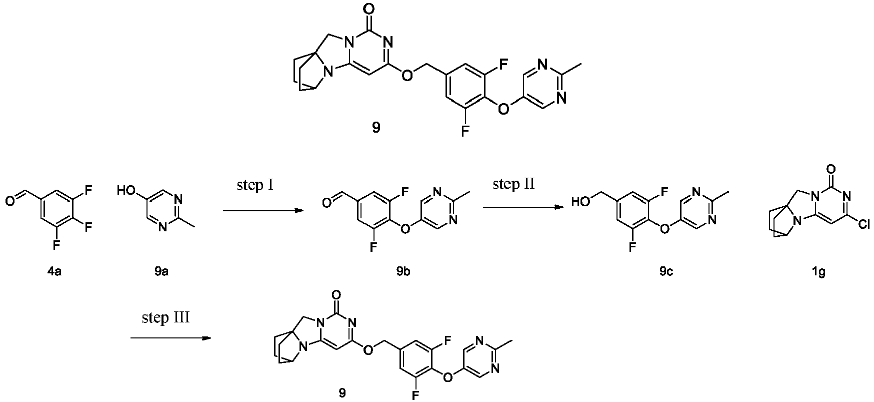

### Step I: Preparation of Compound 9b

At room temperature, 2-methylpyrimidin-5-ol **9a** (0.25 g, 2.27 mmol), 3,4,5-trifluorobenzaldehyde **4a** (0.44 g, 2.72 mmol) and potassium carbonate (0.41 g, 2.95 mmol) were dissolved in 30 mL of *N,N*-dimethylformamide, and stirred and reacted at 90°C for 2 h. After cooled, the reactant was poured into 100 mL of ice water, and extracted with ethyl acetate (50 mL×3). The organic phases were combined, washed with saturated aqueous sodium chloride solution, dried over anhydrous sodium sulfate, and filtered to remove the drying agent. The filtrate was concentrated under reduced pressure, and purified with an eluent system (petroleum ether/ethyl acetate = 10:1) by the silica gel column chromatography to give a yellow solid product **9b** (0.24 g, yield: 34.8 %).

¹H NMR (400 MHz, CDCl₃) δ 9.93 (s, 1H), 8.39 (s, 2H), 7.64 - 7.54 (m, 2H), 2.72 (s, 3H).

### Step II: Preparation of Compound 9c

At room temperature, 3,5-difluoro-4-((2-methylpyrimidin-5-yl)oxy)benzaldehyde **9b** (0.24 g, 0.79 mmol) was dissolved in 50 mL of methanol, to which NaBH**4** (30 mg, 0.79 mmol) was added at 0°C, stirred and reacted at room temperature for 0.5 h, concentrated under reduced pressure, added with water, and extracted with ethyl acetate (100 mL×2). The organic phases were combined, washed with sodium chloride solution, dried over anhydrous sodium sulfate, and filtered to remove the drying agent. The filtrate was concentrated under reduced pressure, and purified with an eluent system (petroleum ether/ethyl acetate = 4:1) by the silica gel column chromatography to give a colorless, oily product **9c** (0.17 g, yield: 85.4 %).

¹H NMR (400 MHz, CDCl₃) δ 8.33 (s, 2H), 7.04 (m, 2H), 4.71 (m, 2H), 2.70 (s, 3H).

### Step III: Preparation of Compound 9

(3,5-Difluoro-4-((2-methylpyrimidin-5-yl)oxy)phenyl)methanol **9c** (53 mg, 0.21 mmol) was dissolved in 5 mL of dry *N,N*-dimethylformamide, to which sodium hydride (60% in mineral oil, 17 mg, 0.42 mmol) was added at 0°C. After the materials were stirred and reacted at room temperature for 20 min, Compound **1g** (50 mg, 0.21 mmol) was added, and stirred and reacted for 1 h. Thereafter, a small amount of water was added to quench the reaction. The reactant was purified with an eluent system (dichloromethane/methanol = 20:1) by the silica gel column chromatography to give a white solid product **9** (18 mg, yield: 19%).

¹H NMR (400 MHz, CDCl₃) δ 8.34 (s, 2H), 7.09 (m, 2H), 5.37 (s, 2H), 5.20 (s, 1H), 4.12 (m, 1H), 3.99 (s, 2H), 2.69 (s, 3H), 2.13 - 2.03 (m, 2H), 1.81 - 1.71 (m, 6H). MS (ESI): m/z 454.0 [M+H]⁺.

### Example 10 Preparation of Compound 10

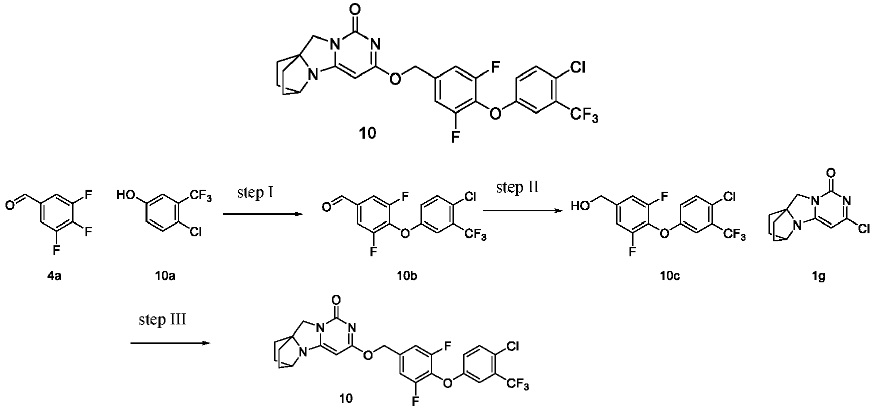

### Step I: Preparation of Compound 10b

At room temperature, 4-chloro-3-(trifluoromethyl)phenol **10a** (0.5 g, 2.54 mmol), 3,4,5-trifluorobenzaldehyde **4a** (0.45 g, 2.8 mmol) and potassium carbonate (0.46 g, 3.3 mmol) were dissolved in 30 mL of *N,N*-dimethylformamide, and stirred and reacted at 90°C for 2 h. After cooled, the reactant was poured into 100 mL of ice water, and extracted with ethyl acetate (50 mL×3). The organic phases were combined, washed with saturated aqueous sodium chloride solution, dried over anhydrous sodium sulfate, and filtered to remove the drying agent. The filtrate was concentrated under reduced pressure, and purified with an eluent system (petroleum ether/ethyl acetate = 10/1) by the silica gel column chromatography to give a yellow solid product **10b** (0.6 g, yield: 70.1 %).

¹H NMR (400 MHz, CDCl₃) δ 9.94 (s, 1H), 7.64 - 7.55 (m, 2H), 7.45 (m, 1H), 7.31 (m, 1H), 7.05 (m, 1H).

### Step II: Preparation of Compound 10c

At room temperature, 4-(4-chloro-3-(trifluoromethyl)phenoxy)-3,5-difluorobenzaldehyde **10b** (0.6 g, 1.78 mmol) was dissolved in 50 mL of methanol, to which NaBH₄ (67 mg, 1.78 mmol) was added at 0°C, stirred and reacted at room temperature for 0.5 h, concentrated under reduced pressure, added with water, and extracted with ethyl acetate (100 mL×2). The organic phases were combined, washed with saturated aqueous sodium chloride solution, dried over anhydrous sodium sulfate, and filtered to remove the drying agent. The filtrate was concentrated under reduced pressure, and purified with an eluent system (petroleum ether/ethyl acetate = 4/1) by the silica gel column chromatography to give a white solid product **10c** (0.28 g, yield: 46.4 %).

¹H NMR (400 MHz, CDCl₃) δ 7.41 (m, 1H), 7.28 (m, 1H), 7.08 - 7.00 (m, 3H), 4.73 (m, 2H), 1.94 (m, 1H).

### Step III: Preparation of Compound 10

(4-(4-Chloro-3-(trifluoromethyl)phenoxy)-3,5-difluorophenyl)methanol **10c** (71 mg, 0.21 mmol) was dissolved in 5 mL of dry *N,N*-dimethylformamide, to which sodium hydride (60% in mineral oil, 17 mg, 0.42 mmol) was added at 0°C. After the materials were stirred and reacted at room temperature for 20 min, Compound **1g** (50 mg, 0.21 mmol) was added, and stirred and reacted for 1 h. Thereafter, a small amount of water was added to quench the reaction. The reactant was purified with an eluent system (dichloromethane/methanol = 20:1) by the silica gel column chromatography to give a white solid product **10** (28 mg, yield: 25%).

¹H NMR (400 MHz, CDCl₃) δ 7.41 (m, 1H), 7.29 (m, 1H), 7.08 (m, 2H), 7.01 (m, 1H), 5.38 (s, 2H), 5.20 (s, 1H), 4.13 (m, 1H), 4.00 (s, 2H), 2.19 - 2.08 (m, 2H), 1.84 - 1.74 (m, 6H). MS (ESI): m/z 539.9 [M+H]⁺.

### Example 11 Preparation of Compound 11

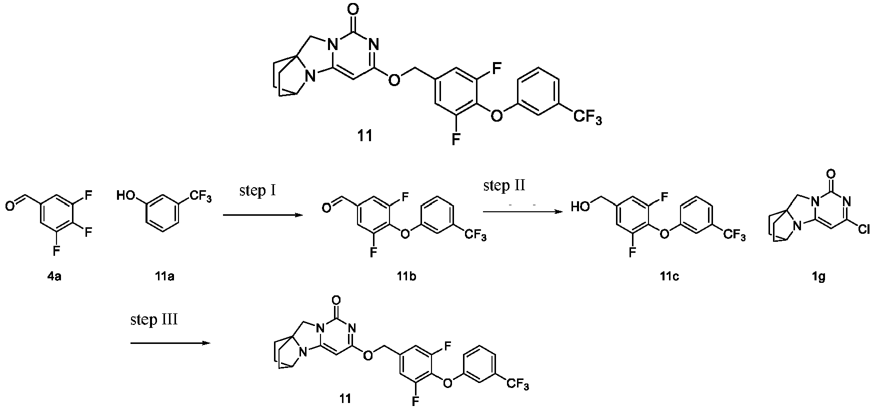

### Step I: Preparation of Compound 11b

At room temperature, 3-(trifluoromethyl)phenol **11a** (1 g, 6.17 mmol), 3,4,5-trifluorobenzaldehyde **4a** (1.09 g, 6.79 mmol) and potassium carbonate (1.1 g, 8.02 mmol) were dissolved in 30 mL of *N,N*-dimethylformamide, and stirred and reacted at 90°C for 2 h. After cooled, the reactant was poured into 100 mL of ice water, and extracted with ethyl acetate (50 mL×3). The organic phases were combined, washed with saturated aqueous sodium chloride solution, dried over anhydrous sodium sulfate, and filtered to remove the drying agent. The filtrate was concentrated under reduced pressure, and purified with an eluent system (petroleum ether/ethyl acetate = 10/1) by the silica gel column chromatography to give a yellow solid product **11b** (1.7 g, yield: 82.5 %).

¹H NMR (400 MHz, CDCl₃) δ 9.94 (s, 1H), 7.63 - 7.55 (m, 2H), 7.46 (m, 1H), 7.39 (m, 1H), 7.21 (s, 1H), 7.13 (m, 1H).

### Step II: Preparation of Compound 11c

At room temperature, 3,5-difluoro-4-(3-(trifluoromethyl)phenoxy)benzaldehyde **11b** (1.7 g, 5.6 mmol) was dissolved in 50 mL of methanol, to which NaBH₄ (213 mg, 5.6 mmol) was added at 0°C, stirred and reacted at room temperature for 0.5 h, concentrated under reduced pressure, added with water, and extracted with ethyl acetate (100 mL×2). The organic phases were combined, washed with saturated aqueous sodium chloride solution, dried over anhydrous sodium sulfate, and filtered to remove the drying agent. The filtrate was concentrated under reduced pressure, and purified with an eluent system (petroleum ether/ethyl acetate = 4/1) by the silica gel column chromatography to give a colorless, oily product **11c** (1.27 g, yield: 74.5 %).

### Step III: Preparation of Compound 11

(3,5-Difluoro-4-(3-(trifluoromethyl)phenoxy)phenyl)methanol **11c** (71 mg, 0.21 mmol) was dissolved in 5 mL of dry *N,N*-dimethylformamide, to which sodium hydride (60% in mineral oil, 17 mg, 0.42 mmol) was added at 0°C. After the materials were stirred and reacted at room temperature for 20 min, Compound **1g** (50 mg, 0.21 mmol) was added, and stirred and reacted for 1 h. Thereafter, a small amount of water was added to quench the reaction. The reactant was purified with an eluent system (dichloromethane/methanol = 20:1) by the silica gel column chromatography to give a white solid product **11** (33 mg, yield: 31%).

¹H NMR (400 MHz, CDCl₃) δ 7.44 (m, 1H), 7.36 (m, 1H), 7.22 (s, 1H), 7.11 (m, 3H), 5.42 (s, 2H), 5.23 (s, 1H), 4.15 (m, 1H), 4.03 (s, 2H), 2.12 (m, 2H), 1.83 - 1.77 (m, 6H). MS (ESI): m/z 505.9 [M+H]⁺.

### Example 12 Preparation of Compound 12

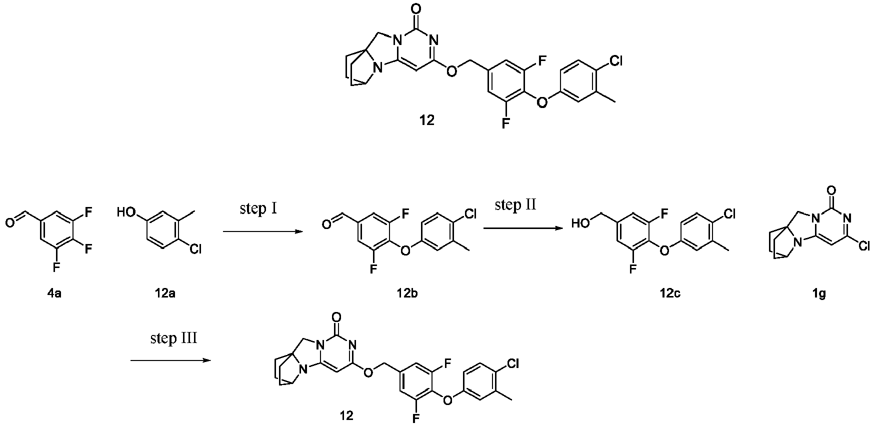

### Step I: Preparation of Compound 12b

At room temperature, 4-chloro-3-methylphenol **12a** (1 g, 7.0 mmol), 3,4,5-trifluorobenzaldehyde **4a** (1.2 g, 7.7 mmol) and potassium carbonate (1.3 g, 9.1 mmol) were dissolved in 30 mL of *N,N*-dimethylformamide, and stirred and reacted at 90°C for 2 h. After cooled, the reactant was poured into 100 mL of ice water, and extracted with ethyl acetate (50 mL×3). The organic phases were combined, washed with saturated aqueous sodium chloride solution, dried over anhydrous sodium sulfate, and filtered to remove the drying agent. The filtrate was concentrated under reduced pressure, and purified with an eluent system (petroleum ether/ethyl acetate = 10/1) by the silica gel column chromatography to give a yellow solid product **12b** (1.2 g, yield: 54.5 %).

¹H NMR (400 MHz, CDCl₃) δ 9.92 (s, 1H), 7.61 - 7.51 (m, 2H), 7.30 - 7.23 (m, 1H), 6.85 (m, 1H), 6.73 (m, 1H), 2.34 (s, 3H).

### Step II: Preparation of Compound 12c

At room temperature, 4-(4-chloro-3-methylphenoxy)-3,5-difluorobenzaldehyde **12b** (1.2 g, 4.2 mmol) was dissolved in 50 mL of methanol, to which NaBH₄ (161 mg, 4.2 mmol) was added at 0°C, stirred and reacted at room temperature for 0.5 h, concentrated under reduced pressure, added with water, and extracted with ethyl acetate (100 mL×2). The organic phases were combined, washed with saturated aqueous sodium chloride solution, dried over anhydrous sodium sulfate, and filtered to remove the drying agent. The filtrate was concentrated under reduced pressure, and purified with an eluent system (petroleum ether/ethyl acetate = 4/1) by the silica gel column chromatography to give a colorless, oily product **12c** (0.89 g, yield: 74.4 %).

### Step III: Preparation of Compound 12

4-(4-Chloro-3-methylphenoxy)-3,5-difluorophenyl)methanol **12c** (59 mg, 0.21 mmol) was dissolved in 5 mL of dry *N,N*-dimethylformamide, to which sodium hydride (60% in mineral oil, 17 mg, 0.42 mmol) was added at 0°C. After the materials were stirred and reacted at room temperature for 20 min, Compound **1g** (50 mg, 0.21 mmol) was added, and stirred and reacted for 1 h. Thereafter, a small amount of water was added to quench the reaction. The reactant was purified with an eluent system (dichloromethane/methanol = 20:1) by the silica gel column chromatography to give a white solid product **12** (29 mg, yield: 31%).

¹H NMR (400 MHz, CDCl₃) δ 7.25 (m, 1H), 7.08 (m, 2H), 6.84 (m, 1H), 6.73 (m, 1H), 5.40 (s, 2H), 5.22 (s, 1H), 4.15 (m, 1H), 4.03 (s, 2H), 2.35 (s, 3H), 2.12 (m, 2H), 1.87 - 1.74 (m, 6H). MS (ESI): m/z 486.0 [M+H]⁺.

### Example 13: Preparation of Compound 13

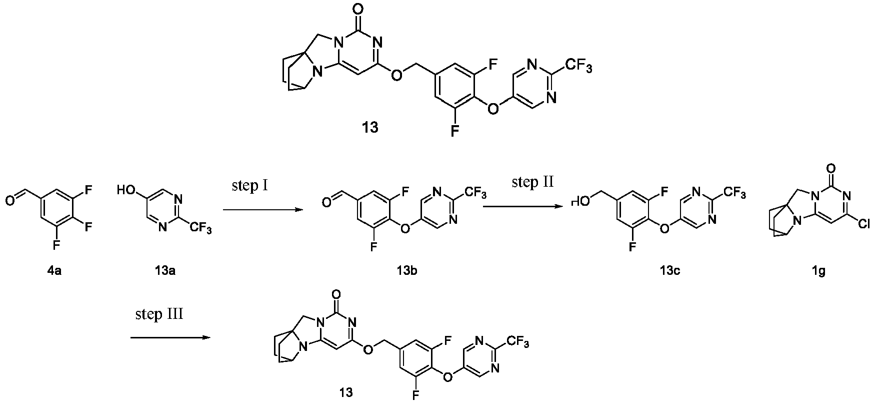

### Step I: Preparation of Compound 13b

At room temperature, 2-(trifluoromethyl)pyrimidin-5-ol **13a** (0.25 g, 1.52 mmol), 3,4,5-trifluorobenzaldehyde **4a** (0.29 g, 1.83 mmol) and potassium carbonate (0.27 g, 1.98 mmol) were dissolved in 20 mL of *N,N*-dimethylformamide, and stirred and reacted at 90°C for 2 h. After cooled, the reactant was poured into 100 mL of ice water, and extracted with ethyl acetate (50 mL×3). The organic phases were combined, washed with saturated aqueous sodium chloride solution, dried over anhydrous sodium sulfate, and filtered to remove the drying agent. The filtrate was concentrated under reduced pressure, and purified with an eluent system (petroleum ether/ethyl acetate = 10/1) by the silica gel column chromatography to give a yellow solid product **13b** (0.24 g, yield: 52.0 %).

¹H NMR (400 MHz, CDCl₃) δ 9.97 (s, 1H), 8.59 (s, 2H), 7.69 - 7.54 (m, 2H).

### Step II: Preparation of Compound 13c

At room temperature, 3,5-difluoro-4-((2-(trifluoromethyl)pyrimidin-5-yl)oxy)benzaldehyde **13b** (0.24 g, 0.79 mmol) was dissolved in 50 mL of methanol, to which NaBH₄ (30 mg, 0.79 mmol) was added at 0°C, stirred and reacted at room temperature for 0.5 h, concentrated under reduced pressure, added with water, and extracted with ethyl acetate (100 mL×2). The organic phases were combined, washed with saturated aqueous sodium chloride solution, dried over anhydrous sodium sulfate, and filtered to remove the drying agent. The filtrate was concentrated under reduced pressure, and purified with an eluent system (petroleum ether/ethyl acetate = 4/1) by the silica gel column chromatography to give a colorless, oily product **13c** (0.12 g, yield: 49.6 %).

¹H NMR (400 MHz, CDCl₃) δ 8.54 (s, 2H), 7.12 (m, 2H), 4.74 (m, 2H), 2.23 (m, 1H).

### Step III: Preparation of Compound 13

(3,5-Difluoro-4-((2-(trifluoromethyl)pyrimidin-5-yl)oxy)phenyl)methanol **13c** (64 mg, 0.21 mmol) was dissolved in 5 mL of dry *N,N*-dimethylformamide, to which sodium hydride (60% in mineral oil, 17 mg, 0.42 mmol) was added at 0°C. After the materials were stirred and reacted at room temperature for 20 min, Compound **1g** (50 mg, 0.21 mmol) was added, and stirred and reacted for 1 h. Thereafter, a small amount of water was added to quench the reaction. The reactant was purified with an eluent system (dichloromethane/methanol = 20:1) by the silica gel column chromatography to give a white solid product **13** (18 mg, yield: 17%).

¹H NMR (400 MHz, CDCl₃) δ 8.58 (s, 2H), 7.17 (m, 2H), 5.43 (s, 2H), 5.24 (s, 1H), 4.16 (m, 1H), 4.03 (s, 2H), 2.13 (m, 2H), 1.88 - 1.76 (m, 6H).

MS (ESI): m/z 508.1 [M+H]⁺.

### Example 14: Preparation of Compound 14

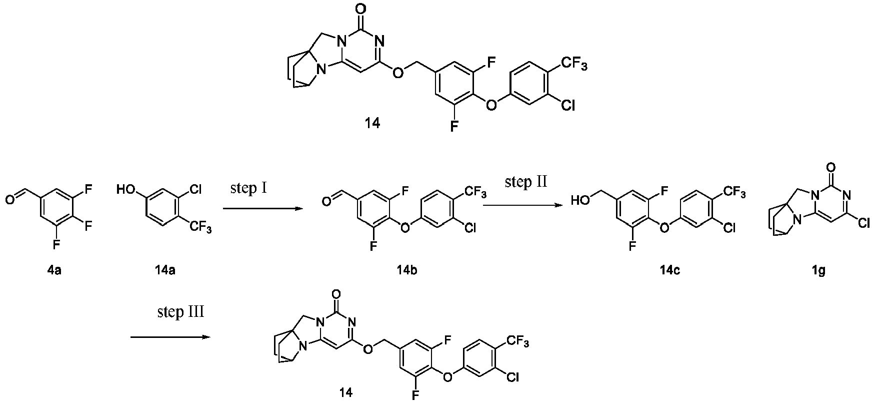

### Step I: Preparation of Compound 14b

At room temperature, 3-chloro-4-(trifluoromethyl)phenol 14a (0.25 g, 1.27 mmol), 3,4,5-trifluorobenzaldehyde 4a (0.22 g, 1.4 mmol) and potassium carbonate (0.23 g, 1.65 mmol) were dissolved in 20 mL of *N,N*-dimethylformamide, and stirred and reacted at 90°C for 2 h. After cooled, the reactant was poured into 100 mL of ice water, and extracted with ethyl acetate (50 mL×3). The organic phases were combined, washed with saturated aqueous sodium chloride solution, dried over anhydrous sodium sulfate, and filtered to remove the drying agent. The filtrate was concentrated under reduced pressure, and purified with an eluent system (petroleum ether/ethyl acetate = 10/1) by the silica gel column chromatography to give a yellow solid product **14b** (0.32 g, yield: 74.0 %).

¹H NMR (400 MHz, CDCl₃) δ 9.95 (s, 1H), 7.69 - 7.56 (m, 3H), 7.10 (m, 1H), 6.92 (m, 1H).

### Step II: Preparation of Compound 14c

At room temperature, 4-(3-chloro-4-(trifluoromethyl)phenoxy)-3,5-difluorobenzaldehyde **14b** (0.32 g, 0.94 mmol) was dissolved in 50 mL of methanol, to which NaBH₄ (36 mg, 0.94 mmol) was added at 0°C, stirred and reacted at room temperature for 0.5 h, concentrated under reduced pressure, added with water, and extracted with ethyl acetate (100 mL×2). The organic phases were combined, washed with saturated aqueous sodium chloride solution, dried over anhydrous sodium sulfate, and filtered to remove the drying agent. The filtrate was concentrated under reduced pressure, and purified with an eluent system (petroleum ether/ethyl acetate = 4/1) by the silica gel column chromatography to give a white solid product **14c** (0.15 g, yield: 47.1 %).

¹H NMR (400 MHz, CDCl₃) δ 7.62 (m, 1H), 7.13 - 7.00 (m, 3H), 6.90 (m, 1H), 4.74 (m, 2H), 1.88 (m, 1H).

### Step III: Preparation of Compound 14

(4-(3-Chloro-4-(trifluoromethyl)phenoxy)-3,5-difluorophenyl)methanol **14c** (71 mg, 0.21 mmol) was dissolved in 5 mL of dry *N,N*-dimethylformamide, to which sodium hydride (60% in mineral oil, 17 mg, 0.42 mmol) was added at 0°C. After the materials were stirred and reacted at room temperature for 20 min, Compound **1g** (50 mg, 0.21 mmol) was added, and stirred and reacted for 1 h. Thereafter, a small amount of water was added to quench the reaction. The reactant was purified with an eluent system (dichloromethane/methanol = 20:1) by the silica gel column chromatography to give a white solid product **14** (12 mg, yield: 10%).

¹H NMR (400 MHz, CDCl₃) δ 7.64 (m, 1H), 7.10 (m, 3H), 6.91 (m, 1H), 5.41 (s, 2H), 5.23 (s, 1H), 4.15 (m, 1H), 4.03 (s, 2H), 2.19 - 2.04 (m, 2H), 1.83 - 1.75 (m, 6H). MS (ESI): m/z 539.9 [M+H]⁺.

### Example 15 Preparation of Compound 15

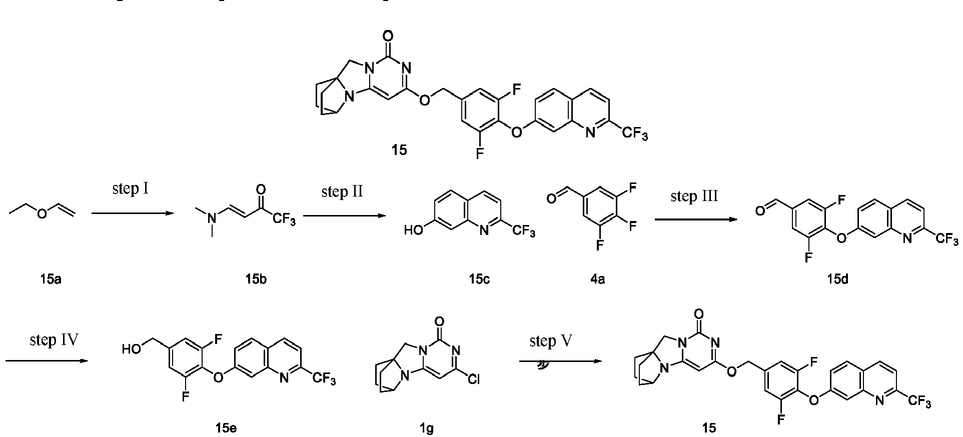

### Step I: Preparation of Compound 15b

Ethoxyethylene **15a** (1 g, 13.9 mmol) and pyridine (1.65 g, 20.9 mmol) were dissolved in 20 mL of dichloromethane, and trifluoroacetic anhydride was added at 0°C under nitrogen protection. The materials were stirred and reacted for 20 min, heated to room temperature, and reacted for 2 h. The reaction mixture was cooled to -20°C, to which dimethylamine was added dropwise within 10 min, heated again to room temperature, and reacted for 2 h. The reaction was quenched with water. The reactant was extracted with dichloromethane (50 mL×3). The organic phases were combined, washed with saturated aqueous sodium chloride solution, dried over anhydrous sodium sulfate, and filtered to remove the drying agent. The filtrate was concentrated under reduced pressure, and purified with an eluent system (petroleum ether/ethyl acetate = 4/1) by the silica gel column chromatography to give a white solid product **15b** (540 mg, yield: 23.1%).

¹H NMR (400 MHz, CDCl₃) δ 7.85 (m, 1H), 5.26 (m, 1H), 3.21 (s, 3H), 2.95 (s, 3H).

### Step II: Preparation of Compound 15c

At room temperature, (E)-4-(dimethylamino)-1,1,1-trifluorobut-3-en-2-one **15b** (0.54 g, 2.98 mmol) was dissolved in 20 mL of dichloroethane, and a solution of Tf₂O in dichloroethane (5 mL) was added dropwise at 0°C, followed by adding a solution of 3-aminophenol in dichloroethane (10 mL). The materials were stirred and reacted at 40°C for 2 h, and filtered to give a white solid product **15c** (275 mg, 57.4%).

¹H NMR (400 MHz, MeOD) δ 8.43 (m, 1H), 7.90 (m, 1H), 7.62 (m, 1H), 7.40 (m, 1H), 7.32 (m, 1H).

### Step III: Preparation of Compound 15d

At room temperature, 2-(trifluoromethyl)quinolin-7-ol **15c** (0.37 g, 1.71 mmol), 3,4,5-trifluorobenzaldehyde **4a** (0.28 g, 1.71 mmol) and potassium carbonate (0.71 g, 5.13 mmol) were dissolved in 20 mL of *N,N*-dimethylformamide, and stirred and reacted at 90°C for 2 h. After cooled, the reactant was poured into 100 mL of ice water, and extracted with ethyl acetate (50 mL×3). The organic phases were combined, washed with saturated aqueous sodium chloride solution, dried over anhydrous sodium sulfate, and filtered to remove the drying agent. The filtrate was concentrated under reduced pressure, and purified with an eluent system (petroleum ether/ethyl acetate = 5/1) by the silica gel column chromatography to give a white solid product **15d** (310 mg, 51.5%).

¹H NMR (400 MHz, CDCl₃) δ 9.97 (s, 1H), 8.36 (m, 1H), 7.96 (m, 1H), 7.69 (m, 1H), 7.62 (m, 3H), 7.40 (s, 1H).

### Step IV: Preparation of Compound 15e

At room temperature, 3,5-difluoro-4-((2-(trifluoromethyl)quinolin-7-yl)oxy)benzaldehyde **15d** (0.31 g, 0.88 mmol) was dissolved in 20 mL of methanol, to which NaBH₄ (33 mg, 0.88 mmol) was added at 0°C, stirred and reacted at room temperature for 0.5 h, concentrated under reduced pressure, added with water, and extracted with ethyl acetate (100 mL×2). The organic phases were combined, washed with saturated aqueous sodium chloride solution, dried over anhydrous sodium sulfate, and filtered to remove the drying agent. The filtrate was concentrated under reduced pressure, and purified with an eluent system (petroleum ether/ethyl acetate = 4/1) by the silica gel column chromatography to give a colorless, oily product **15e** (230 mg, 74.6%).

### Step V: Preparation of Compound 15

(3,5-Difluoro-4-((2-(trifluoromethyl)quinolin-7-yl)oxy)phenyl)methanol **15e** (75 mg, 0.21 mmol) was dissolved in 5 mL of dry *N,N*-dimethylformamide, to which sodium hydride (60% in mineral oil, 17 mg, 0.42 mmol) was added at 0°C. After the materials were stirred and reacted at room temperature for 20 min, Compound **1g** (50 mg, 0.21 mmol) was added, and stirred and reacted for 1 h. Thereafter, a small amount of water was added to quench the reaction. and purified with an eluent system (dichloromethane/methanol = 20:1) by the silica gel column chromatography to give a white solid product **15** (18 mg, yield: 17%).

¹H NMR (400 MHz, CDCl₃) δ 8.33 (m, 1H), 7.92 (m, 1H), 7.65 (m, 1H), 7.60 (m, 1H), 7.36 (s, 1H), 7.13 (m, 2H), 5.40 (s, 2H), 5.23 (s, 1H), 4.14 (m, 1H), 4.02 (s, 2H), 2.11 (m, 2H), 1.79 (m, 6H). MS (ESI): m/z 557.0 [M+H]⁺.

### Example 16 Preparation of Compound 16

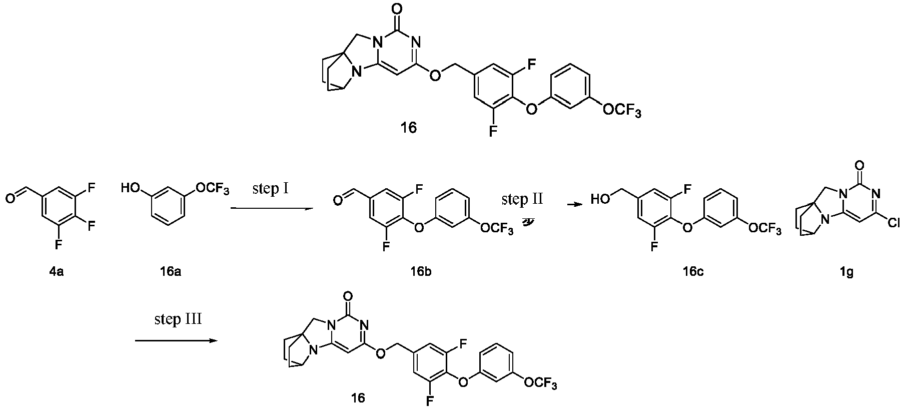

### Step I: Preparation of Compound 16b

At room temperature, 3-(trifluoromethoxy)phenol **16a** (0.50 g, 2.8 mmol), 3,4,5-trifluorobenzaldehyde **4a** (0.5 g, 2.8 mmol) and potassium carbonate (0.5 g, 3.64 mmol) were dissolved in 30 mL of *N,N*-dimethylformamide, and stirred and reacted at 90°C for 2 h. After cooled, the reactant was poured into 100 mL of ice water, and extracted with ethyl acetate (50 mL×3). The organic phases were combined, washed with saturated aqueous sodium chloride solution, dried over anhydrous sodium sulfate, and filtered to remove the drying agent. The filtrate was concentrated under reduced pressure, and purified with an eluent system (petroleum ether/ethyl acetate = 10/1) by the silica gel column chromatography to give a white solid product **16b** (0.73 g, yield: 81.4 %).

¹H NMR (400 MHz, CDCl₃) δ 9.94 (s, 1H), 7.64 - 7.54 (m, 2H), 7.34 (m, 1H), 7.00 (m, 1H), 6.87 (m, 2H).

### Step II: Preparation of Compound 16c

At room temperature, 4-(3-(trifluoromethoxy)phenoxy)-3,5-difluorobenzaldehyde **16b** (0.73 g, 2.28 mmol) was dissolved in 50 mL of methanol, to which NaBH₄ (86 mg, 2.28 mmol) was added at 0°C, stirred and reacted at room temperature for 0.5 h, concentrated under reduced pressure, added with water, and extracted with ethyl acetate (100 mL×2). The organic phases were combined, washed with saturated aqueous sodium chloride solution, dried over anhydrous sodium sulfate, and filtered to remove the drying agent. The filtrate was concentrated under reduced pressure, and purified with an eluent system (petroleum ether/ethyl acetate = 4/1) by the silica gel column chromatography to give a colorless, oily product **16c** (0.57g, yield: 71.2 %).

¹H NMR (400 MHz, CDCl₃) δ 7.30 (m, 1H), 7.06 (m, 2H), 6.94 (m, 1H), 6.85 (m, 1H), 6.81 (s, 1H), 4.72 (m, 2H), 1.94 (m, 1H).

### Step III: Preparation of Compound 16

4-(3-(Trifluoromethoxy)phenoxy)-3,5-difluorophenyl)methanol **16c** (67 mg, 0.21 mmol) was dissolved in 5 mL of dry *N,N*-dimethylformamide, to which sodium hydride (60% in mineral oil, 17 mg, 0.42 mmol) was added at 0°C. After the materials were stirred and reacted at room temperature for 20 min, Compound **1g** (50 mg, 0.21 mmol) was added, and stirred and reacted for 1 h. Thereafter, a small amount of water was added to quench the reaction. The reactant was purified with an eluent system (dichloromethane/methanol = 20:1) by the silica gel column chromatography to give a white solid product **16** (25 mg, yield: 23%).

¹H NMR (400 MHz, CDCl₃) δ 7.29 (m, 1H), 7.07 (m, 2H), 6.93 (m, 1H), 6.83 (m, 2H), 5.38 (s, 2H), 5.20 (s, 1H), 4.12 (m, 1H), 4.00 (s, 2H), 2.08 (m, 2H), 1.83 - 1.73 (m, 6H). MS (ESI): m/z 522.0 [M+H]⁺.

### Example 17 Preparation of Compound 17

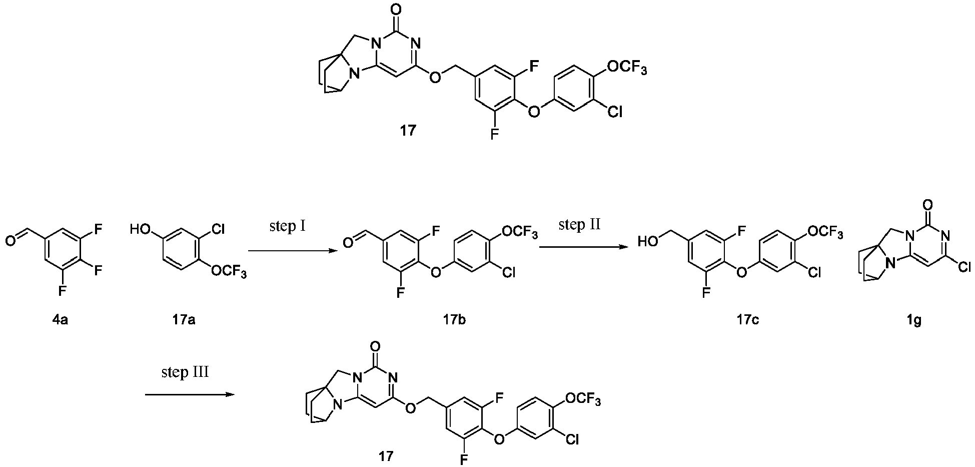

### Step I: Preparation of Compound 17b

At room temperature, 3-chloro-4-(trifluoromethoxy)phenol **17a** (0.50 g, 2.34 mmol), 3,4,5-trifluorobenzaldehyde **4a** (0.41 g, 2.58 mmol) and potassium carbonate (0.42 g, 3.04 mmol) were dissolved in 20 mL of *N,N*-dimethylformamide, and stirred and reacted at 90°C for 2 h. After cooled, the reactant was poured into 100 mL of ice water, and extracted with ethyl acetate (50 mL×3). The organic phases were combined, washed with saturated aqueous sodium chloride solution, dried over anhydrous sodium sulfate, and filtered to remove the drying agent. The filtrate was concentrated under reduced pressure, and purified with an eluent system (petroleum ether/ethyl acetate = 10/1) by the silica gel column chromatography to give a yellow solid product **17b** (0.62 g, yield: 74.4 %).

¹H NMR (400 MHz, CDCl₃) δ 9.94 (s, 1H), 7.63 - 7.54 (m, 2H), 7.29 (m, 1H), 7.07 (m, 1H), 6.90 (m, 1H).

### Step II: Preparation of Compound 17c

At room temperature, 4-(3-chloro-4-(trifluoromethoxy)phenoxy)-3,5-difluorobenzaldehyde **17b** (0.62 g, 1.94 mmol) was dissolved in 50 mL of methanol, to which NaBH₄ (62 mg, 1.94 mmol) was added at 0°C, stirred and reacted at room temperature for 0.5 h, concentrated under reduced pressure, added with water, and extracted with ethyl acetate (100 mL×2). The organic phases were combined, washed with saturated aqueous sodium chloride solution, dried over anhydrous sodium sulfate, and filtered to remove the drying agent. The filtrate was concentrated under reduced pressure, and purified with an eluent system (petroleum ether/ethyl acetate = 4/1) by the silica gel column chromatography to give a colorless, oily product **17c** (0.53 g, yield: 77.0 %).

¹H NMR (400 MHz, CDCl₃) δ 7.25 (m, 1H), 7.06 (m, 2H), 7.01 (m, 1H), 6.87 (m, 1H), 4.72 (s, 2H), 2.04 (m, 1H).

### Step III: Preparation of Compound 17

(4-(3-Chloro-4-(trifluoromethoxy)phenoxy)-3,5-difluorophenyl)methanol **17c** (75 mg, 0.21 mmol) was dissolved in 5 mL of dry *N,N*-dimethylformamide, to which sodium hydride (60% in mineral oil, 17 mg, 0.42 mmol) was added at 0°C. After the materials were stirred and reacted at room temperature for 20 min, Compound **1g** (50 mg, 0.21 mmol) was added, and stirred and reacted for 1 h. Thereafter, a small amount of water was added to quench the reaction.and purified with an eluent system (dichloromethane/methanol = 20:1) by the silica gel column chromatography to give a white solid product 17 (23 mg, yield: 20%).

¹H NMR (400 MHz, CDCl₃) δ 7.23 (s, 1H), 7.09 (m, 2H), 7.03 (m, 1H), 6.87 (m, 1H), 5.39 (s, 2H), 5.21 (s, 1H), 4.13 (m, 1H), 4.01 (s, 2H), 2.10 (m, 2H), 1.83 - 1.71 (m, 6H). MS (ESI): m/z 555.9 [M+H]⁺.

### Example 18 Preparation of Compound 18

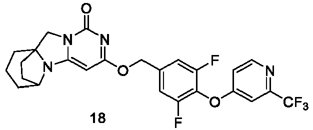

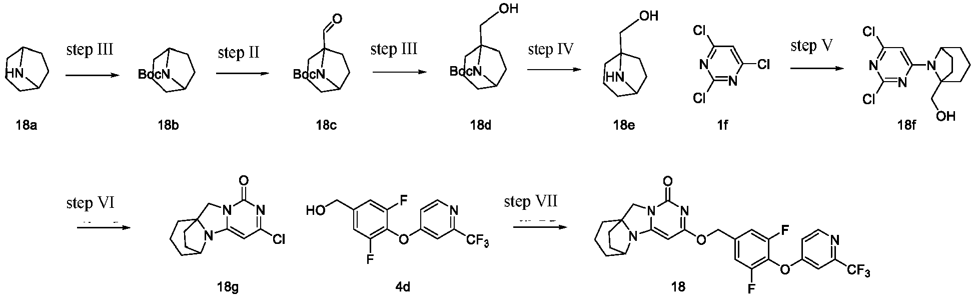

### Step I: Preparation of Compound 18b

At room temperature, 8-azabicyclo[3.2.1]octane **18a** (5 g, 33.9 mmol) and triethylamine (5.2 g, 50.8 mmol) were dissolved in 300 mL of dichloromethane, and (Boc)₂O (11.1 g, 50.8 mmol) was added. The materials were stirred and reacted for 4 h, and extracted with dichloromethane (100 mL×3). The organic phases were combined, washed with saturated aqueous sodium chloride solution, dried over anhydrous sodium sulfate, and filtered to remove the drying agent. The filtrate was concentrated under reduced pressure, and purified with an eluent system (petroleum ether/ethyl acetate = 10/1) by the silica gel column chromatography to give a colorless liquid product **18b** (9.4 g, 100%).

¹H NMR (400 MHz, CDCl₃) δ 4.14 (m, 2H), 1.97 - 1.86 (m, 2H), 1.81 - 1.53 (m, 6H), 1.46 (s, 9H), 1.41 - 1.35 (m, 2H).

### Step II: Preparation of Compound 18c

At room temperature, tert-butyl 8-azabicyclo[3.2.1]octane-8-carboxylate **18b** (7.2 g, 34.1 mmol) and TMEDA (4.7 g, 40.9 mmol) were dissolved in 150 mL of dry ether, and s-BuLi (1.3 M in hexane, 31.5 mL, 40.9 mmol) was added dropwise at -65°C under nitrogen protection. After stirring and reacting at -65°C for 30 min, a solution of methyl formate (2.45 g, 40.9 mmol) in ether (20 mL) was added dropwise. After stirring and reacting at -65°C for 30 min, the reactant was heated to 0°C, stirred and reacted for 2 h, added with a saturated aqueous ammonium chloride solution (100 mL), and extracted with ether (50 mL×3). The organic phases were combined, dried over anhydrous sodium sulfate, and filtered to remove the drying agent. The filtrate was concentrated under reduced pressure, and purified with an eluent system (petroleum ether/ethyl acetate = 10:1) by the silica gel column chromatography to give a colorless, liquid product **18c** (3.13 g, 38.4%).

¹H NMR (400 MHz, CDCl₃) δ 9.45 (s, 1H), 4.24 (m, 1H), 2.15 (s, 1H), 1.97 (m, 1H), 1.85 (m, 2H), 1.82 - 1.60 (m, 6H), 1.45 (s, 9H).

### Step III: Preparation of Compound 18d

At room temperature, tert-butyl 1-formyl-8-azabicyclo[3.2.1]octane-8-carboxylate **18c** (3.13 g, 13.1 mmol) was dissolved in 100 mL of dry methanol, and KBH₄ (1.1 g, 19.6 mmol) was added in batch at 0°C. The materials were stirred and reacted at room temperature for 1 h, added with 40 mL of water, and extracted with dichloromethane (60 mL×3). The organic phases were combined, washed with saturated aqueous sodium chloride solution, dried over anhydrous sodium sulfate, and filtered to remove the drying agent. The filtrate was concentrated under reduced pressure, and purified with an eluent system (petroleum ether/ethyl acetate = 10/1) by the silica gel column chromatography to give a colorless liquid product **18d** (3.45 g, 100%).

¹H NMR (400 MHz, CDCl₃) δ 5.37 (m, 1H), 4.21 (m, 1H), 3.72 - 3.49 (m, 2H), 2.00 - 1.73 (m, 4H), 1.61 (m, 6H), 1.46 (s, 9H).

### Step IV: Preparation of Compound 18e

At room temperature, tert-butyl 1-(hydroxymethyl)-8-azabicyclo[3.2.1]octane-8-carboxylate **18d** (3.13 g, 13.1 mmol) was dissolved in 60 mL of a mixed solvent of HCl/ethanol, stirred and reacted for 4 h, and concentrated under reduced pressure to give a yellow solid product **18e** (2.45 g, 100%).

¹H NMR (400 MHz, DMSO) δ 8.94 (s, 2H), 5.55 (s, 1H), 3.80 (m, 1H), 3.58 - 3.48 (m, 2H), 2.04 (m, 1H), 1.86 - 1.40 (m, 9H).

### Step V: Preparation of Compound 18f

At room temperature, (8-azabicyclo[3.2.1]oct-1-yl)methanol **18e** (2.45 g, 17.35 mmol), 2,4,6-trichloropyrimidine (3.49 g, 19.1 mmol) and DIPEA (0.42 g, 52.05 mmol) were dissolved in 100 mL of acetonitrile, stirred and reacted overnight, concentrated under reduced pressure, and purified with an eluent system (petroleum ether/ethyl acetate = 4:1) by the silica gel column chromatography to give a white solid product **18f** (1 g, 20.1%).

¹H NMR (400 MHz, CDCl₃) δ 6.41 (s, 1H), 5.43 (m, 1H), 4.30 (m, 1H), 3.83 (m, 1H), 3.72 (m, 1H), 2.13 - 1.52 (m, 10H).

### Step VI: Preparation of Compound 18g

**18f** (1 g, 3.1 mmol) and triethylamine (0.94 g, 1.3 mmol) were dissolved in 50 mL of dry dichloromethane, and MsCl (0.4 g, 3.4 mmol) was added dropwise at 0°C. After stirring and reacting at 0°C for 1 h, the reactant was concentrated under reduced pressure to give a colorless, oily intermediate (1.1 g, 100%). This coarse product and potassium carbonate (1.28 g, 9.3 mmol) were dissolved in 60 mL of a mixed solution of dioxane/water (1/1), stirred and reacted at 90°C overnight, concentrated under reduced pressure, and purified with an eluent system (dichloromethane/methanol = 20/1) by the silica gel column chromatography to give a white solid product **18g** (0.4 g, 51%).

¹H NMR (400 MHz, ¹H NMR (400 MHz, CDCl₃) δ 5.66 (s, 1H), 4.25 (m, 1H), 4.11 (m, 1H), 3.70 (m, 1H), 2.05 - 1.88 (m, 4H), 1.85 - 1.71 (m, 6H).

### Step VII: Preparation of Compound 18

(3,5-Difluoro-4-((2-(trifluoromethyl)pyridin-4-yl)oxy)phenyl)methanol **4d** (92 mg, 0.3 mmol) was dissolved in 5 mL of dry DMF, to which sodium hydride (60% in mineral oil, 17 mg, 0.4 mmol) was added at 0°C. After the materials were stirred and reacted at room temperature for 20 min, Compound **18g** (50 mg, 0.2 mmol) was added, and stirred and reacted for 1 h. Thereafter, a small amount of water was added to quench the reaction. The reactant was purified with an eluent system (dichloromethane/methanol = 20:1) by the silica gel column chromatography to give a white solid product **18** (49 mg, yield: 47%).

¹H NMR (400 MHz, CDCl₃) δ 8.60 (m, 1H), 7.26 (s, 1H), 7.13 (m, 2H), 6.98 (m, 1H), 5.41 (s, 2H), 5.19 (s, 1H), 4.24 (m, 1H), 4.07 (m, 1H), 3.70 (m, 1H), 2.05 - 1.87 (m, 3H), 1.73 (m, 7H). MS (ESI): m/z 521.2 [M+H]⁺.

### Example 19 Preparation of Compound 19

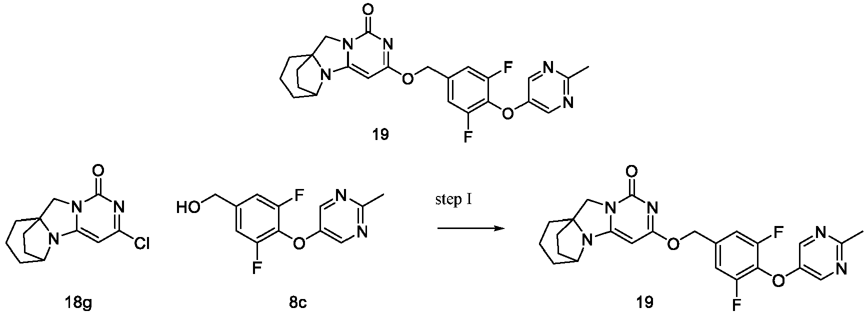

### Step I: Preparation of Compound 19

(3,5-Difluoro-4-((2-methylpyridin-4-yl)oxy)phenyl)methanol **8c** (53 mg, 0.21 mmol) was dissolved in 5 mL of dry DMF, to which sodium hydride (60% in mineral oil, 17 mg, 0.4 mmol) was added at 0°C. After the materials were stirred and reacted at room temperature for 20 min, Compound **18g** (50 mg, 0.2 mmol) was added, and stirred and reacted for 1 h. Thereafter, a small amount of water was added to quench the reaction. The reactant was purified with an eluent system (dichloromethane/methanol = 20:1) by the silica gel column chromatography to give a white solid product **19** (52 mg, yield: 55.5%).

¹H NMR (400 MHz, CDCl₃) δ 8.38 (s, 2H), 7.10 (m, 2H), 5.41 (s, 2H), 5.20 (s, 1H), 4.23 (m, 1H), 4.06 (m, 1H), 3.68 (m, 1H), 2.70 (s, 3H), 2.00 - 1.90 (m, 3H), 1.85 - 1.69 (m, 7H). MS (ESI): m/z 468.2 [M+H]⁺.

### Example 20 Preparation of Compound 20

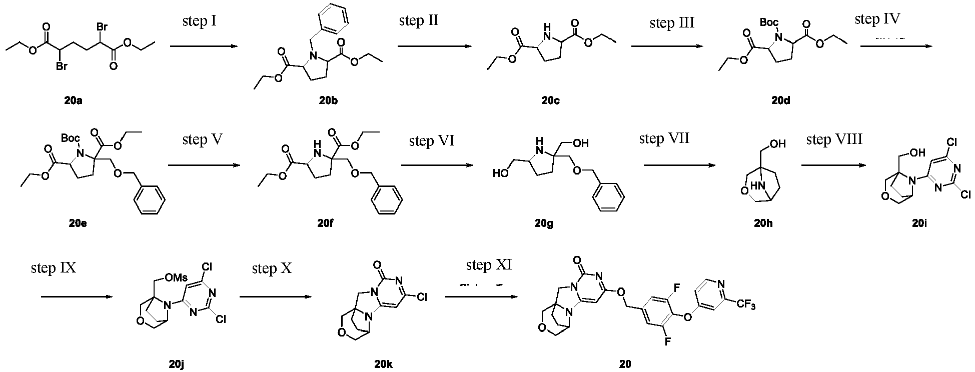

### Step I: Preparation of Compound 20b

At room temperature, the compound diethyl meso-2,5-dibromoadipate (10.0 g, 27.8 mmol) and potassium carbonate (4.60 g, 33.3 mmol) were dissolved in toluene and water (200 mL, v/v = 4:1), and phenylmethylamine (2.99 g, 27.8 mmol) was added. The reaction mixture was heated to 80°C and reacted for 24 h. The reaction mixture was cooled to room temperature, concentrated under reduced pressure to remove most of the solvent, added with water, and extracted with ethyl acetate (80 mL × 3). The organic layers were combined, dried over anhydrous sodium sulfate, and filtered to remove the drying agent. The filtrate was concentrated under reduced pressure, and purified with an eluent system (petroleum ether/ethyl acetate = 10/1) by the silica gel column chromatography to give an oily product **20b** (6.6 g, 77.7%).

¹H NMR (400 MHz, DMSO) δ 7.31 - 7.18 (m, 5H), 3.96 - 3.86 (m, 4H), 3.83 (s, 2H), 3.38 (m, 2H), 2.08 - 1.98 (m, 2H), 1.94 - 1.83 (m, 2H), 1.08 (m, 6H).

### Step II: Preparation of Compound 20c

At room temperature, the compound diethyl N-benzyl-2,5-pyrrole dicarboxylate (6.6 g, 21.6 mmol) was dissolved in methanol (100 mL), and palladium on carbon (20%, 1.3 g) was added. The reaction mixture was reacted for 7 h at room temperature under the H₂ condition. The reaction mixture was filtered, and the filtrate was concentrated under reduced pressure to give a colorless, oily Compound **20c** (4.4 g, 94.4%).

¹H NMR (400 MHz, CDC13) δ 4.23 - 4.11 (m, 4H), 3.81 - 3.71 (m, 2H), 2.18 - 2.06 (m, 2H), 1.97 - 1.83 (m, 2H), 1.26 (m, 6H).

### Step III: Preparation of Compound 20d

At room temperature, the compound diethyl 2,5-pyrrolidinecarboxylate (4.4 g, 19.5 mmol) was dissolved in toluene, and di-tert-butyl dicarbonate (5.35 g, 24.5 mmol) was added. The reaction mixture was heated to 95°C and reacted for 4 h. The reaction mixture was cooled to room temperature, concentrated under reduced pressure, and purified with an eluent system (petroleum ether/ethyl acetate = 5/1) by the silica gel column chromatography to give a colorless, oily product **20d** (4.3 g, 70.0 %).

¹H NMR (400 MHz, CDC13) δ 4.38 (s, 1H), 4.27 (s, 1H), 4.24 - 4.14 (m, 4H), 2.16 (m, 4H), 1.42 (s, 9H), 1.31 - 1.21 (m, 6H).

### Step IV: Preparation of Compound 20e

At room temperature, the compound diethyl N-tert-butoxycarbonyl-2,5-pyrrole dicarboxylate (2.7 g, 8.6 mmol) was dissolved in dry tetrahydrofuran (80 mL). A solution of lithium diisopropylamide (2 M, 8.2 ml, 16.3 mmol) in tetrahydrofuran was added dropwise at - 78°C under nitrogen protection. Benzylchloromethyl ether (1.49g, 9.5 mmol) was added. The mixture was stirred and reacted at -78°C for 1 h, and heated to the room temperature and reacted for 2 h. To the reaction mixture, 100 mL of saturated aqueous ammonium chloride solution was added, and extracted with ethyl acetate (50 mL×3). The organic phases were combined, dried over anhydrous sodium sulfate, and filtered to remove the drying agent. The filtrate was concentrated under reduced pressure, and purified with an eluent system (petroleum ether/ethyl acetate = 5/1) by the silica gel column chromatography to give a yellow, oily product **20e** (2.3 g, 61.4%).

¹H NMR (400 MHz, CDC13) δ 7.41 - 7.20 (m, 5H), 4.71 - 4.52 (m, 2H), 4.25 - 4.07 (m, 4H), 2.49 (m, 1H), 2.28 (m, 1H), 2.15 - 2.01 (m, 2H), 1.37 (s, 9H), 1.29 - 1.17 (m, 6H).

### Step V: Preparation of Compound 20f

At room temperature, the compound diethyl N-tert-butoxycarbonyl-2,5-(2-phenoxymethyl)pyrrole dicarboxylate was dissolved in dioxane hydrochloride (4N, 20 mL), and stirred at room temperature for 2 h. The reaction mixture was concentrated under reduced pressure to give a brown oily coarse Compound **20f** (1.44 g, 50.0%) for direct use in the next step without purification.

¹H NMR (400 MHz, CDC13) δ 7.39 - 7.27 (m, 5H), 4.60 - 4.46 (m, 2H), 3.71 (m, 1H), 3.55 (m, 2H), 3.44 - 3.27 (m, 3H), 1.89 - 1.76 (m, 1H), 1.76 - 1.45 (m, 3H).

### Step VI: Preparation of Compound 20g

Dry tetrahydrofuran (40 mL) was added to a reaction flask, and lithium aluminum hydride (0.33 g, 8.6 mmol) was added under ice bath and nitrogen protection. Ten minutes later, a solution of [6-(benzyloxymethyl)-6-(hydroxymethyl)-2-piperidinyl]methanol in tetrahydrofuran was added dropwise, and then moved to room temperature and reacted for 1 h. The reaction was performed under ice-bath conditions again, and sodium hydroxide solution (2M, 2 mL) was added to produce a white solid. The white solid was filtered and washed with tetrahydrofuran. The filtrate was dried over anhydrous sodium sulfate, and filtered to remove the drying agent. The filtrate was concentrated under reduced pressure to give a yellowish oily coarse Compound **20g** (1.0 g, 92.5 %) for direct use in the next step without purification.

¹H NMR (400 MHz, CDC13) δ 7.39 - 7.27 (m, 5H), 4.57 - 4.46 (m, 2H), 3.71 (m, 1H), 3.55 (m, 2H), 3.44 - 3.31 (m, 3H), 1.88 - 1.78 (m, 1H), 1.75 - 1.58 (m, 2H), 1.52 (m,1H).

### Step VII: Preparation of Compound 20h

At room temperature, the compound (2-((benzyloxy)methyl)pyrrolidine-2,5-diyl)dimethanol (1.0 g, 3.98 mmol) was dissolved in methanesulfonic acid under argon protection. The reaction mixture was heated to 140°C and reacted for 8 h. After cooling to room temperature, the reaction solution was poured into ice water (20 ml), 24 ml of 50% sodium hydroxide was added at 0°C. After concentrated under reduced pressure, the mixture was dissolved in methanol and filtered, and the filtrate was concentrated under reduced pressure to give a yellowish oily coarse target Compound **20h** (0.3 g, 52.8%).

### Step VIII: Preparation of Compound 20i

At room temperature, the compound 3-oxa-8-azabicyclo[3.2.1]oct-5-ylmethanol (0.3 g, 2.1 mmol), 2,4,6-trichloropyrimidine (0.46 g, 2.5 mmol), and diisopropylethylamine (0.81 g, 6.3 mmol) were dissolved in acetonitrile (20 ml), and the reaction mixture was stirred at room temperature overnight. The reaction mixture was concentrated under reduced pressure, and purified with an eluent system (petroleum ether/ethyl acetate = 2:1) by the silica gel column chromatography to give a white solid compound **20i** (0.15g, 24.8%).

¹H NMR (400 MHz, CDC13) δ 6.39 (s, 1H), 5.25 (m, 1H), 4.22 (m, 1H), 3.98 - 3.76 (m, 3H), 3.67 (m, 3H), 2.23 - 2.14 (m, 1H), 2.14 - 1.96 (m, 2H), 1.78 (m, 1H).

### Steps IX & X: Preparation of Compound 20k

At room temperature, the compound (8-(2,6-dichloropyrimidin-4-yl)-3-oxa-8-azabicyclo[3.2.1]oct-1-yl)methanol (0.15, 24.8%) was dissolved in dry dichloromethane, triethylamine (158 mg, 1.56 mmol) was added, and placed in an ice bath. Methanesulfonyl chloride (66 mg, 0.57 mmol) was slowly added. The reaction mixture was reacted in the ice bath for 1 h. The reaction mixture was concentrated under reduced pressure to give a white solid compound, which was used directly in the next step without purification. The coarse product and potassium carbonate (215 mg, 1.56 mmol) were dissolved in dioxane and water (40 mL, 1/1). The reaction mixture was reacted at room temperature overnight. The reaction mixture was concentrated under reduced pressure, and purified with an eluent system (dichloromethane/methanol = 20/1) by the silica gel column chromatography to give a white solid Compound **20k** (40 mg, 30.8%).

¹H NMR (400 MHz, CDC13) δ 5.72 (s, 1H), 4.29 (m, 1H), 4.08 (m, 1H), 3.97 (m, 1H), 3.77 (m, 4H), 2.46 - 2.27 (m, 1H), 2.14 (m, 1H), 1.96 (m, 1H), 1.77 (m, 1H).

### Step XI: Preparation of Compound 20

At room temperature, the compound (3,5-difluoro-4-((2-(trifluoromethyl)pyridin-4-yl)oxy)phenyl)methanol (58, 0.19 mmol) was dissolved in dry N,N-dimethylformamide (5 mL), and sodium hydride (60% in mineral oil, 13 mg, 0.32 mmol) was added in an ice bath. 7-Chloro-3,4-dihydro-1H-4,11a-ethanopyrimido[6',1':2,3]imidazo[5,1-c][1,4]azinyl-9(11H)-one (40 mg, 0.16 mol) was added 15 min later, and reacted at room temperature for 1 h. The reaction mixture was quenched by adding ice water, and extracted with ethyl acetate (20 ml×3). The organic layers were combined, dried over anhydrous sodium sulfate, and filtered to remove the drying agent. The filtrate was concentrated under reduced pressure, and purified with an eluent system (dichloromethane/methanol = 20/1) by the silica gel column chromatography to give a white solid compound **20** (35 mg, 35.3 %).

¹H NMR (600 MHz, CDCl₃) δ 8.61 (m, 1H), 7.26 (s, 1H), 7.14 (m, 2H), 6.99 (m, 1H), 5.43 (s, 2H), 5.22 (s, 1H), 4.26 (m, 1H), 4.00 (m, 1H), 3.93 (m, 1H), 3.78 - 3.68 (m, 4H), 2.33 - 2.26 (m, 1H), 2.08 (m, 1H), 1.99 - 1.92 (m, 1H), 1.73 (m, 1H). m/z 523.0 [M+H]⁺.

### Biological Evaluation

The bioactivity of a compound could be determined using any suitable assay as well as tissue and *in vivo* model for determining the activity of a compound as an LpPLA2 inhibitor.

### (1) Recombinant human Lp-PLA2 assay (rhLp-PLA2), also referred to as PED6 assay

PED6 was a fluorescently-labeled phospholipid that could be purchased dorectly from Invitogene or Molecular Probes. It had a fluorescence-quenching *p*-nitrophenyl group at the Sn3 position and a Bodipy fluorescein (FL) group at the sn2 position. Once cleaved by the Lp-PLA2 enzyme, it would release the FL group, resulting in enhanced fluorescence. However, the Lp-PLA2 inhibitor could prevent occurrence of such cleavage, so that no enhanced fluorescence could be observed.

Assay Method: The compound to be tested (as shown in Table 1) was mixed with a DMSO solution in a volume ratio of 1:3, and diluted to prepare a source plate of a 384-well microplate. Then 0.01 µl of the compound was transferred via an ECHO liquid dispenser from the source plate to a 384-well Greiner 784076 plate, and 5 µl of a buffer consisting of 50 mM HEPES, pH7.4, 150 mM NaCl, and 1 mM CHAPS (the buffer solution containing a recombinant human Lp-PLA2 enzyme at a concentration of 4 nM or 110 pM) was added to each well of the plate. The plate was centrifuged at 500 rpm for 10 seconds. After a 30-min pre-incubation, 5 µl of the above-mentioned buffer was added to a 384-well Greiner 784076 plate, the plate was centrifuged at 500 rpm for 10 seconds. After the plate was incubated at room temperature for 20 min in a dark place, the fluorescence intensity was read at ex 480/em 540 with a ViewLux microplate imager, and the XL fitting model in Excel was used to perform the curve analysis and QC analysis to calculate pIC50. The results were listed in Table 1.

**Table 1**

| Compound No. | rhLp-PLA2 (pIC₅₀) |
|---|---|
| 1 | 8.6 |
| 2 | 9.1 |
| 3 | 9.3 |
| 4 | 10.4 |
| 5 | 10.5 |
| 6 | 10.3 |
| 7 | 10.4 |
| 8 | 10.5 |
| 9 | 10.0 |
| 10 | 10.2 |
| 11 | 10.1 |
| 12 | 10.1 |
| 13 | 9.6 |
| 14 | 10.1 |
| 16 | 10.0 |
| 17 | 10.1 |
| 18 | 10.0 |
| 19 | 9.5 |
| Positive compound Rilapladib | 8.9 |

### (2) Assay of Lp-PLA2 in human plasma (also referred to as Thio-PAF assay)

The human plasma assay was conducted using the sulphatide analog of PAF (phosphatidylcholine). After hydrolysis, it would generate phospholipids containing free sulfhydryl groups, which would be subjected to Michael addition with CPM to generate fluorescence-enhancing maleimide. Continuous quantitative analysis of thiol could be conducted by detecting the fluorescence intensity. This assay could be used to detect the inhibitory activity of the Lp-PLA2 inhibitor on the Lp-PLA2 enzyme in human plasma.

Assay Method: The compound to be tested (as shown in Table 2) was mixed with a DMSO solution in a volume ratio of (1:3), and diluted to prepare a source plate of a 384-well microplate. Then 0.01 µl of the compound was transferred via an ECHO liquid dispenser from the source plate to a 384-well Greiner 784076 plate, and 8 µl of pre-aliquoted and frozen mixed human plasma was then added. The plate was centrifuged at 500 rpm for 10 seconds. After a 30-min pre-incubation, 2 µl of a substrate solution, and a buffer containing 2.5 mM 2-thio-PAF (a solution in ethanol), 32 µM CPM (a solution in DMSO) and 3.2 mM N-ethylmaleimide (NEM) (a buffer solution consisting of 50 mM HEPES, pH7.4, 150 mM NaCl, 1 mM CHAPS) was added by a BRAVO liquid handling station to a 384-well Greiner 784076 low-volume plate. Two minutes later, the reaction was quenched with 5 µl of 5% trifluoroacetic acid. After the plate was incubated at room temperature for 40 min in a dark place, the fluorescence intensity was read at ex 380/ em 485 with an Envision microplate reader, and the XL fitting model in Excel was used to perform the curve analysis and QC analysis to calculate pIC50. The results were listed in Table 2.

**Table 2**

| Compound No. | Thio-PAF (pIC₅₀) |
|---|---|
| 1 | 7.0 |
| 2 | 7.6 |
| 3 | 7.8 |
| 4 | 8.4 |
| 5 | 8.2 |
| 6 | 8.1 |
| 7 | 8.5 |
| 8 | 8.1 |
| 9 | 7.9 |
| 10 | 8.1 |
| 11 | 7.9 |
| 12 | 8.0 |
| 13 | 7.7 |
| 14 | 8.1 |
| 16 | 7.9 |
| 17 | 8.0 |
| 18 | 8.1 |
| 19 | 7.8 |
| Positive compound Rilapladib | 7.8 |

## Claims

1. A compound represented by formula (I): 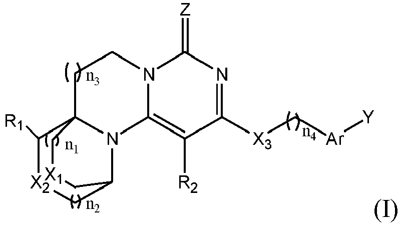
or a pharmaceutically acceptable salt thereof,
wherein:
n₁, n₂, n₃, and n₄ are each independently 0, 1, or 2;
R¹ and R² are each independently selected from: -H, hydroxyl, cyano, halogen, alkyl, deuteroalkyl, hydroxyalkyl, haloalkyl, cycloalkyl, and alkoxyl;
X₁, X₂, and X₃ are each independently selected from: alkylene, -O-, -S-, or -NR'-;
R' is selected from: -H, alkyl, deuteroalkyl, or cycloalkyl;
Ar is arylene or heteroarylene; and hydrogen atoms in the arylene or heteroarylene are optionally substituted by one or more substituents that are each independently selected from: halogen, alkyl, haloalkyl, alkoxyl, haloalkoxyl, deuteroalkyl, deuteroalkoxyl, hydroxyl, hydroxyalkyl, cyano, amino, monoalkyl- or dialkyl-substituted amino, nitro, carboxyl, aldehyde, cycloalkyl, heterocyclyl, aryl, or heteroaryl;
Y is -H, halogen, alkyl, haloalkyl, haloalkoxyl, cycloalkyl, alkoxyl, deuteroalkyl, deuteroalkoxyl, -OAr', -SAr', -NH-Ar', -NMe-Ar', -NR", or -R‴-Ar';
Ar' is selected from aryl or heteroaryl; and hydrogen atoms in the aryl or heteroaryl are optionally substituted by one or more substituents that are each independently selected from: halogen, alkyl, haloalkyl, alkoxyl, hydroxyl, hydroxyalkyl, haloalkoxyl, deuteroalkyl, deuteroalkoxyl, cyano, amino, nitro, carboxyl, aldehyde, cycloalkyl, heterocyclyl, aryl, or heteroaryl;
R" is alkyl;
R‴ is alkylene; and
Z is O or S;
optionally, wherein:
n₁, n₂, n₃, and n₄ are each independently 0, 1, or 2;
R¹ and R² are each independently selected from: -H, hydroxyl, cyano, halogen, alkyl, deuteroalkyl, hydroxyalkyl, haloalkyl, cycloalkyl, and alkoxyl;
X₁, X₂, and X₃ are each independently selected from: alkylene, -O-, -S-, or -NR'-;
R' is selected from: -H, alkyl, deuteroalkyl, or cycloalkyl;
Ar is arylene or heteroarylene; and hydrogen atoms in the arylene or heteroarylene are optionally substituted by one or more substituents that are each independently selected from: halogen, alkyl, haloalkyl, alkoxyl, haloalkoxyl, hydroxyl, hydroxyalkyl, cyano, amino, monoalkyl- or dialkyl-substituted amino, nitro, carboxyl, aldehyde, cycloalkyl, heterocyclyl, aryl, or heteroaryl;
Y is -H, halogen, alkyl, haloalkyl, haloalkoxyl, cycloalkyl, alkoxyl, -OAr', -SAr', -NH-Ar', -NMe-Ar', -NR", or -R‴-Ar';
Ar' is selected from aryl or heteroaryl; and hydrogen atoms in the aryl or heteroaryl are optionally substituted by one or more substituents that are each independently selected from: halogen, alkyl, haloalkyl, alkoxyl, hydroxyl, hydroxyalkyl, haloalkoxyl, cyano, amino, nitro, carboxyl, aldehyde, cycloalkyl, heterocyclyl, aryl, or heteroaryl;
R" is alkyl;
R‴ is alkylene; and
Z is O or S.

2. The compound of formula (I), or the pharmaceutically acceptable salt thereof according to claim 1, wherein
halogen atoms in the "halogen", "haloalkyl", and "haloalkoxyl" are each independently selected from F, Cl, Br, or I;
optionally, alkyls in the "alkyl", "deuteroalkyl", "deuteroalkoxyl", "hydroxyalkyl", "haloalkyl", "haloalkoxyl", "alkoxyl", and "monoalkyl- or dialkyl-substituted amino" are each independently C₁-C₁₀ linear or branched alkyl; optionally, C₁-C₇ linear or branched alkyl; optionally, C₁-C₄ linear or branched alkyl; optionally, selected from methyl, ethyl, n-propyl, isopropyl, n-butyl, isobutyl, tert-butyl, sec-butyl, n-pentyl, 1-methylbutyl, 2-methylbutyl, 3-methylbutyl, isopentyl, 1-ethylpropyl, neopentyl, n-hexyl, 1-methylpentyl, 2-methylpentyl, 3-methylpentyl, isohexyl, 1,1-dimethylbutyl, 2,2-dimethylbutyl, 3,3-dimethylbutyl, 1,2-dimethylbutyl, 1,3-dimethylbutyl, 2,3-dimethylbutyl, 2-ethylbutyl, n-heptyl, 2-methylhexyl, 3-methylhexyl, 2,2-dimethylpentyl, 3,3-dimethylpentyl, 2,3-dimethylpentyl, 2,4-dimethylpentyl, 3-ethylpentyl, or 2,2,3-trimethylbutyl;
optionally, the "alkylenes" are each independently C₁-C₁₀ linear or branched alkylene; optionally C₁-C₇ linear or branched alkylene; optionally, C₁-C₅ linear or branched alkylene; and optionally selected from methylene, ethylene, n-propylidene, isopropylidene, n-butylidene, isobutylidene, tert-butylidene, sec-butylidene, n-pentylidene, 1-methylbutylidene, 2-methylbutylidene, 3-methylbutylidene, isopentylidene, 1-ethylpropylidene, neopentylidene, n-hexylidene, 1-methylpentylidene, 2-methylpentylidene, 3-methylpentylidene, isohexylidene, 1,1-dimethylbutylidene, 2,2-dimethylbutylidene, 3,3-dimethylbutylidene, 1,2-dimethylbutylidene, 1,3-dimethylbutylidene, 2,3-dimethylbutylidene, 2-ethylbutylidene, n-heptylidene, 2-methylhexylidene, 3-methylhexylidene, 2,2-dimethylpentylidene, 3,3-dimethylpentylidene, 2,3-dimethylpentylidene, 2,4-dimethylpentylidene, 3-ethylpentylidene, or 2,2,3-trimethylbutylidene;
optionally, the "cycloalkyl" is C₃-C₁₀ monocyclic or bicyclic cycloalkyl, optionally C₃-C₇ monocyclic cycloalkyl, and optionally cyclopropyl, cyclobutyl, cyclopentyl, cyclohexyl, or cycloheptyl;
optionally, the "heterocyclyl" is 3- to 10-membered non-aromatic heterocyclic ring containing 1, 2, or 3 heteroatoms selected from N, O, and S on the ring, optionally, the heterocyclic ring is a 3- to 10-membered non-aromatic ring containing 1 or 2 heteroatoms selected from N and O on the ring; optionally, the heterocyclic ring is a 3- to 6-membered non-aromatic ring containing 1 or 2 heteroatoms selected from N and O on the ring; optionally, the heterocyclic ring is a 3- to 10-membered non-aromatic ring containing 1 or 2 heteroatoms selected from N and S on the ring; and optionally, the heterocyclic ring is a 3- to 6-membered non-aromatic ring containing 1 or 2 heteroatoms selected from N and S on the ring;
optionally, the "aryl" is 6- to 10-membered aryl, optionally phenyl or naphthyl, and optionally phenyl, 1-naphthyl, or 2-naphthyl;
optionally, the "arylene" is 6- to 10-membered arylene, and optionally phenylene or naphthylene;
optionally, the "heteroaryl" is a 5- to 10-membered heteroaromatic ring containing 1 to 3 heteroatoms selected from N, O, and S on the ring, and optionally a 5- to 10-membered heteroaromatic ring containing 1 to 2 heteroatoms selected from N, O, and S on the ring; optionally, the heteroaromatic ring is selected from a pyridine ring, a pyrrole ring, a pyrimidine ring, a pyrazine ring, a pyridazine ring, a thiophene ring, and a furan ring; optionally, the heteroaryl is selected from pyridin-2-yl, pyridin-3-yl, pyridin-4-yl, pyridazin-3-yl, pyridazin-4-yl, pyrimidin-2-yl, pyrimidin-4-yl, pyrimidin-5-yl, pyrazin-2-yl, pyrazin-3-yl, indolyl, isoindolyl, indazolyl, indolizinyl, purinyl, quinolizinyl, quinolinyl, isoquinolinyl, cinolinyl , phthalazinyl, naphthyridinyl, quinazolinyl, quinoxalinyl, thieno[2,3-b]furyl, furo[3,2-b]-pyranyl, pyrido[2,3-d]oxazinyl, pyrazolo[4,3-d]oxazolyl, imidazo[4,5-d]thiazolyl, pyrazino[2,3-d]pyridazinyl, imidazo[2,1-b]thiazolyl, imidazo[1,2-b][1,2,4]triazinyl, benzothienyl, benzoxazolyl, benzimidazolyl, benzothiazolyl, benzoxepinyl, benzoxazinyl, benzofuranyl, benzotriazolyl, pyrrolo[2,3-b]pyridyl, pyrrolo[3,2-c]pyridyl, pyrrolo[3,2-b]pyridyl, imidazo[4,5-b]pyridyl, imidazo[4,5-c]pyridyl, pyrazolo[4,3-d]pyridyl, pyrazolo[4,3-c]pyridyl, pyrazolo[3,4-c]pyridyl, pyrazolo[3,4-d]pyridyl, pyrazolo[3,4-b]pyridyl, imidazo[1,2-a]pyridyl, pyrazolo[1,5-a]pyridyl, pyrrolo[1,2-b]pyridazinyl, imidazo[1,2-c]pyrimidinyl, pyrido[3,2-d]pyrimidinyl, pyrido[4,3-d]pyrimidinyl, pyrido[3,4-d]pyrimidinyl, pyrido[2,3-d]pyrimidinyl, pyrido[2,3-b]pyrazinyl, pyrido[3,4-b]pyrazinyl, pyrimido[5,4-d]pyrimidinyl, pyrazolo[2,3-b]pyrazinyl, and pyrimido[4,5-d]pyrimidinyl, and optionally pyridin-2-yl, pyridin-3-yl, pyridin-4-yl, pyrimidin-2-yl, pyrimidin-4-yl, or pyrimidin-5-yl; and
optionally, the "heteroarylene" is a 5- to 10-membered heteroarylene ring containing 1 to 3 heteroatoms selected from N, O, and S on the ring, and optionally a 5- to 10-membered heteroarylene ring containing 1 to 2 heteroatoms selected from N, O, and S on the ring; and optionally, the heteroarylene ring is selected from a pyridylidene ring, a pyrrylidene ring, a pyrimidylidene ring, a pyrazinylidene ring, a pyridazinylidene ring, a thienylidene ring, a furylidene ring.

3. The compound of formula (I), or the pharmaceutically acceptable salt thereof according to claim 1 or 2, wherein
n₁, n₂, n₃, and n₄ are each independently 0, 1, or 2; optionally, n₁ is 0, optionally, n₂ is 0 or 1, optionally, n₃ is 0, optionally, n₄ is 1;
optionally, R₁, R₂ are each independently selected from: -H, F, Cl, Br, hydroxyl, cyano, C₁-C₇ alkyl (methyl, ethyl, n-propyl, isopropyl, n-butyl, isobutyl, tert-butyl, sec-butyl, n-pentyl, 1-methylbutyl, 2-methylbutyl, 3-methylbutyl, isopentyl, 1-ethylpropyl, neopentyl, n-hexyl, 1-methylpentyl, 2-methylpentyl, 3-methylpentyl, isohexyl, 1,1-dimethylbutyl, 2,2-dimethylbutyl, 3,3-dimethylbutyl, 1,2-dimethylbutyl, 1,3-dimethylbutyl, 2,3-dimethylbutyl, 2-ethylbutyl, n-heptyl, 2-methylhexyl, 3-methylhexyl, 2,2-dimethylpentyl, 3,3-dimethylpentyl, 2,3-dimethylpentyl, 2,4-dimethylpentyl, 3-ethylpentyl, or 2,2,3-trimethylbutyl), C₁-C₃ deuteroalkyl(-CD₃, -C₂D₅, -C₃D₇), cyclopropanyl, cyclobutanyl, and cyclopentyl; optionally, R₁ is -H, and optionally, R₂ is -H;
optionally, X₁, X₂, and X₃ are each independently selected from: C₁-C₇ alkylene (optionally, -CH₂-, ethylene, n-propylidene, isopropylidene, n-butylidene, or isobutylidene), -O-, -S-, or - NR'-; optionally, X₁ is -CH₂-; optionally, X₂ is selected from -CH₂- or -O-; optionally, X₃ is -O-;
optionally, R' is selected from-H, C₁-C₇ alkyl (methyl, ethyl, n-propyl, isopropyl, n-butyl, isobutyl, tert-butyl, sec-butyl, n-pentyl, 1-methylbutyl, 2-methylbutyl, 3-methylbutyl, isopentyl, 1-ethylpropyl, neopentyl, n-hexyl, 1-methylpentyl, 2-methylpentyl, 3-methylpentyl, isohexyl, 1,1-dimethylbutyl, 2,2-dimethylbutyl, 3,3-dimethylbutyl, 1,2-dimethylbutyl, 1,3-dimethylbutyl, 2,3-dimethylbutyl, 2-ethylbutyl, n-heptyl, 2-methylhexyl, 3-methylhexyl, 2,2-dimethylpentyl, 3,3-dimethylpentyl, 2,3-dimethylpentyl, 2,4-dimethylpentyl, 3-ethylpentyl, or 2,2,3-trimethylbutyl), deuteroalkyl (optionally -CD₃, -C₂D₅, -C₃D₇), or C₃-C₆ cycloalkyl (cyclopropanyl, cyclobutanyl, and cyclopentyl, cyclohexyl);
optionally, Ar is phenylene or pyridyl, and a hydrogen atom in the phenylene or pyridyl is optionally substituted by 1, 2, or 3 substituents that are each independently selected from: F, Cl, Br, I, -CN, -Me, -C₂H₅, cyclopropyl, -CD₃, -OMe, or -OCF₃; optionally, Ar is phenylene, and a hydrogen atom in the phenylene is optionally substituted by 2 substituents that are F;
optionally, Y is -H, -F, -Cl, -Br, methyl, ethyl, n-propyl, isoproyl, -CD₃, -CF₃, -CH₂CF₃, - OCF₃, -OCHF₂, cyclopropyl, -cyclobutyl, -cyclopentyl, -OCH₃, -OC₂H₅, -OC₃H₇, or -OAr'; optionally, Y is -H or -OAr';
optionally, Ar' is selected from phenyl, pyridyl, pyrimidinyl, or quinolinyl, and hydrogen atoms in the phenyl, pyridyl, pyrimidinyl or quinolinyl ring are each independently optionally substituted with 1, 2 or 3 substituents that are each independently selected from: F, Cl, Br, -CN, C₁-C₇ alkyl (optionally selected from methyl, ethyl, n-propyl, isopropyl, n-butyl, isobutyl, tert-butyl, sec-butyl, n-pentyl, 1-methylbutyl, 2-methylbutyl, 3-methylbutyl, isopentyl, 1-ethylpropyl, neopentyl, n-hexyl, 1-methylpentyl, 2-methylpentyl, 3-methylpentyl, isohexyl, 1,1-dimethylbutyl, 2,2-dimethylbutyl, 3,3-dimethylbutyl, 1,2-dimethylbutyl, 1,3-dimethylbutyl, 2,3-dimethylbutyl, 2-ethylbutyl, n-heptyl, 2-methylhexyl, 3-methylhexyl, 2,2-dimethylpentyl, 3,3-dimethylpentyl, 2,3-dimethylpentyl, 2,4-dimethylpentyl, 3-ethylpentyl, or 2,2,3-trimethylbutyl;), -CD₃, C₁-C₆haloalkyl, -OCH₃, -OC₂H₇, -OC₃H₇, C₁-C₆ haloalkoxyl, or C₃-C₆ cycloalkyl (optionally cyclopropanyl, cyclobutanyl, cyclopentyl, or cyclohexyl); optionally, Ar' is selected from phenyl, pyridin-3-yl, or pyridin-4-yl, or pyrimidin-5-yl, optionally, the Ar' is substituted by 1 or 2 substituents that are selected from Cl, -CH₃, -CF₃, or -OCF₃; and
optionally, Z is O.

4. The compound of formula (I), or the pharmaceutically acceptable salt thereof according to any one of claims 1 to 3, wherein the compound of formula (I) is selected from the following compounds:

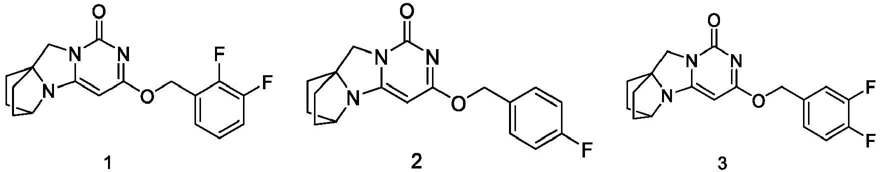

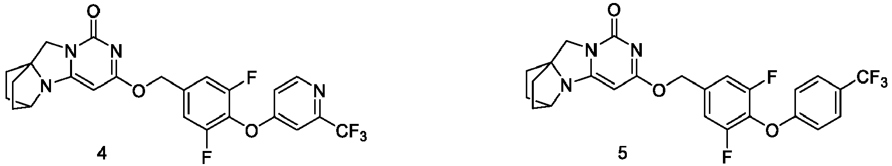

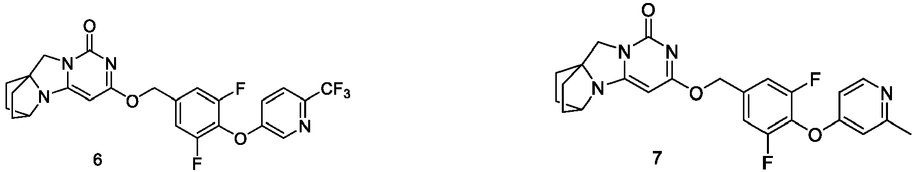

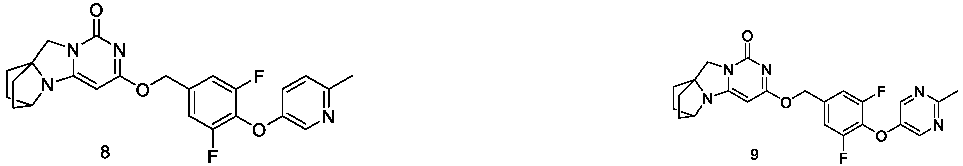

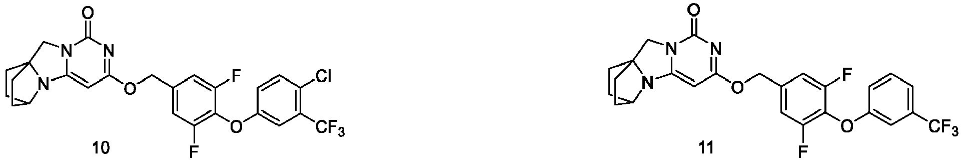

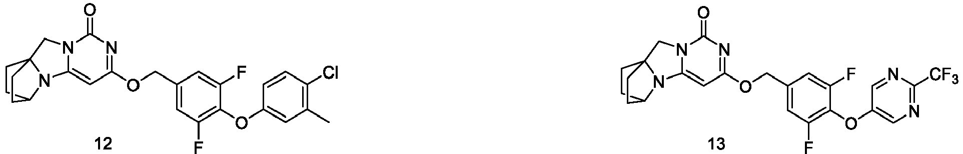

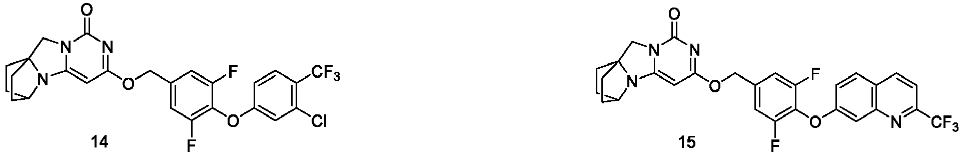

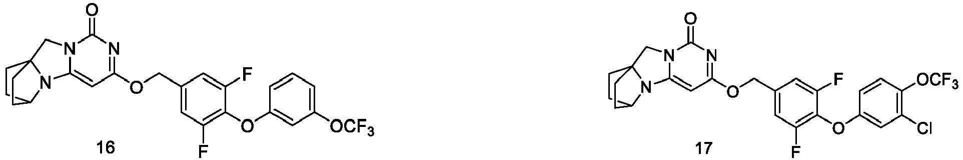

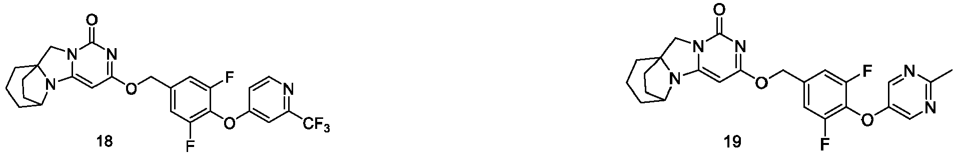

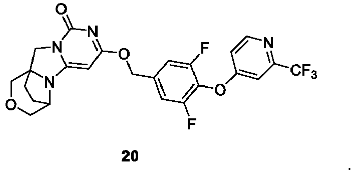

5. The compound of formula (I), or the pharmaceutically acceptable salt thereof according to any one of claims 1 to 4, wherein the pharmaceutically acceptable salt includes an anionic salt and a cationic salt of the compound of formula (I);
optionally, the pharmaceutically acceptable salt includes an alkali metal salt, an alkaline earth metal salt, and an ammonium salt of the compound of formula (I); optionally, the alkali metal includes sodium, potassium, lithium, and cesium, and the alkaline earth metal includes magnesium, calcium, and strontium;
optionally, the pharmaceutically acceptable salt includes a salt formed by the compound of formula (I) and an organic base;
optionally, the organic base includes trialkylamine, pyridine, quinoline, piperidine, imidazole, picoline, dimethylaminopyridine, dimethylaniline, N-alkylmorpholine, 1,5-diazabicyclo[4.3.0]nonene-5, 1,8-diazabicyclo[5.4.0]undecene-7, and 1,4-diazabicyclo[2.2.2]octane; optionally, the trialkylamine includes trimethylamine, triethylamine, and N-ethyldiisopropylamine; and optionally, the N-alkylmorpholine includes N-methylmorpholine;
optionally, the pharmaceutically acceptable salt includes a salt formed by the compound of formula (I) and an acid; and
optionally, the acid includes an inorganic acid and an organic acid; optionally, the inorganic acid includes hydrochloric acid, hydrobromic acid, hydroiodic acid, sulfuric acid, nitric acid, phosphoric acid, carbonic acid; optionally, the organic acid includes formic acid, acetic acid, propionic acid, oxalic acid, malonic acid, succinic acid, fumaric acid, maleic acid, lactic acid, malic acid, citric acid, citric acid, tartaric acid, carbonic acid, picric acid, methanesulfonic acid, ethanesulfonic acid, *p*-toluenesulfonic acid, glutamic acid, and pamoic acid.

6. A method for preparing a compound of formula (I), or a pharmaceutically acceptable salt thereof according to any one of claims 1 to 5, comprising the following synthetic route: 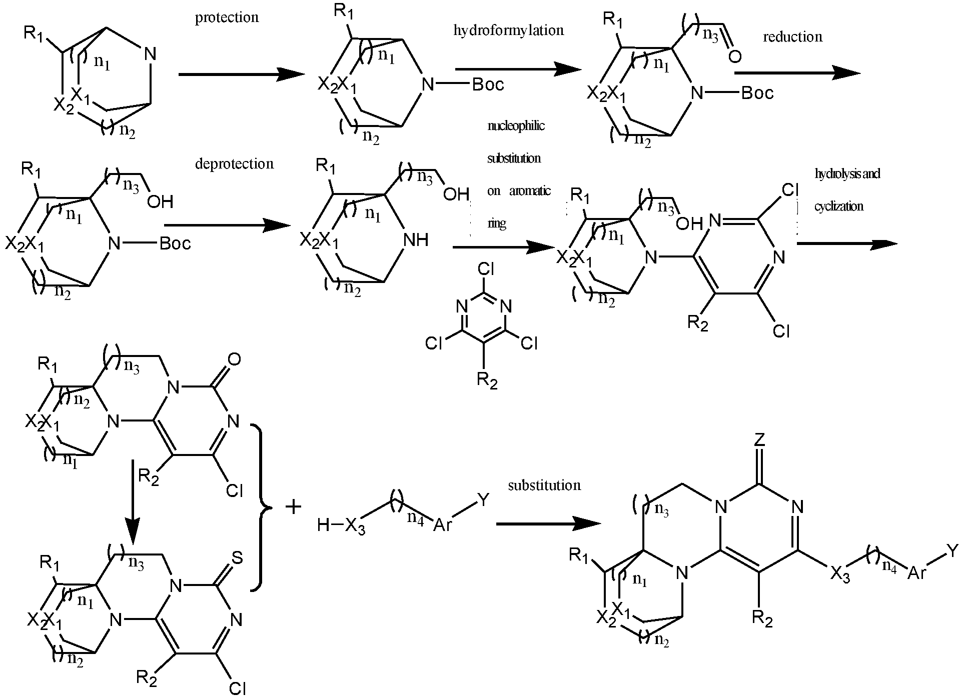
in respective formulas, n₁, n₂, n₃, R₁, R₂, X₁, X₂, X₃, Z, Ar, and Y are as defined in any one of claims 1 to 5;
optionally, when X₂ is -O- and n₃ is 0, the following synthetic route is further adopted:

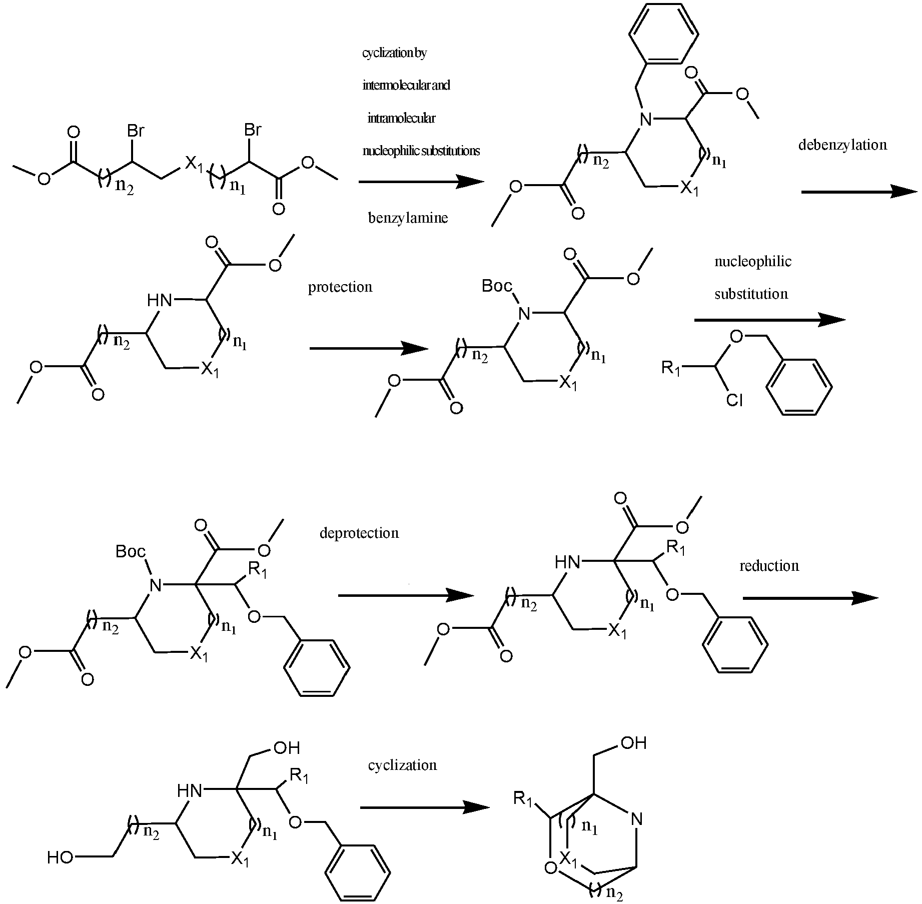

7. A pharmaceutical composition, comprising one or more of a compound of formula (I), or a pharmaceutically acceptable salt thereof according to any one of claims 1 to 5, and optionally a pharmaceutically acceptable carrier.

8. The pharmaceutical composition according to claim 7, wherein a dosage form of the pharmaceutical composition includes an oral preparation, a rectally administered preparation, and a parenteral administered preparation;
optionally, the oral preparation includes a solid preparation and a liquid preparation,
optionally, the solid preparation includes tablets, powders, granules, and capsules;
optionally, the liquid preparation includes aqueous or oily suspensions, and syrups; and
optionally, the parenteral administered preparation includes solutions for injection, and aqueous or oily suspensions.

9. Use of a compound of formula (I) or a pharmaceutically acceptable salt thereof according to any one of claims 1 to 5, or a pharmaceutical composition according to claim 7 or 8 in the preparation of a Lp-PLA2 inhibitor.

10. Use of a compound of formula (I) or a pharmaceutically acceptable salt thereof according to any one of claims 1 to 5, or a pharmaceutical composition according to claim 7 or 8 in the preparation of a medicament for treating a neurodegenerative-related disease;
optionally, the neurodegenerative-related disease includes Alzheimer's disease (AD), glaucoma, and age-related macular degeneration (AMD).

11. Use of a compound of formula (I) or a pharmaceutically acceptable salt thereof according to any one of claims 1 to 5, or a pharmaceutical composition according to claim 7 or 8 in the preparation of a medicament for treating cardiovascular disease, diabetic macular edema (DME), or prostatic disease; and
optionally, the cardiovascular disease includes atherosclerosis.
